# EUROPEAN PATENT APPLICATION

(11) **EP 3 643 761 A1**
(43) Date of publication of application: **29.04.2020**
(21) Application number: 18202590.8
(22) Date of filing: 25.10.2018
(51) Int. Cl.: C09K 11/06, H01L 51/00, H05B 33/20, C07D 495/04

(54) **COMPOSITION, ORGANIC ELECTROLUMINESCENCE DEVICE MATERIAL, COMPOSITION FILM, ORGANIC ELECTROLUMINESCENCE DEVICE, AND ELECTRONIC DEVICE**

(71) Applicant: Idemitsu Kosan Co., Ltd., Chiyoda-ku, Tokyo 100-8321 (JP)
(72) Inventor: Wolleb, Heinz, 4232 Fehren (CH); Boufflet, Pierre, 4055 Basel (CH); Mizuki, Yumiko, 4054 Basel (CH); Groarke, Michelle, 4125 Riehen (CH); Kawamura, Masahiro, 4057 Basel (CH)
(74) Representative: Hollah, Dorothee

(57) **Abstract**

A composition comprising
a compound of formula (1) 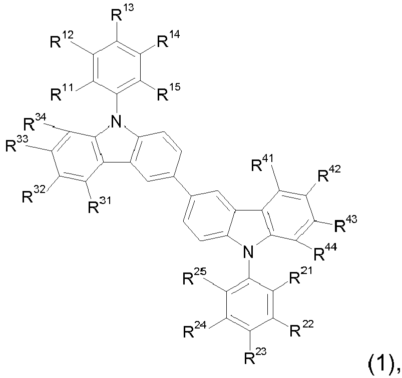 and
a compound of formula (2) 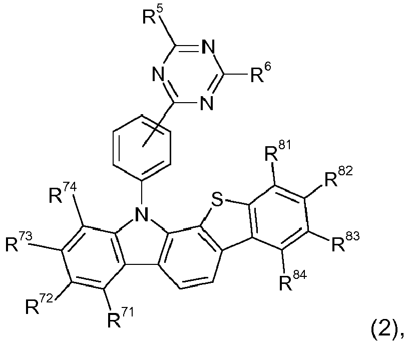 a material for an organic electroluminescence device (also referred to as an organic electroluminescence device material) comprising said composition, a composition film comprising said composition, an organic electroluminescence device (organic EL device) comprising said composition, and an electronic device comprising said organic electroluminescence device.

## Description

The present invention relates to a composition, a material for an organic electroluminescence device (also referred to as an organic electroluminescence device material), a composition film, an organic electroluminescence device (organic EL device) comprising the composition, and an electronic device comprising the organic EL device.

US 2017/0117488, US 2017/0170408, US 2017/0213968 and US 2017/0207396 disclose an organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by a specific biscarbazole derivative containing an aryl group, and a second host compound is represented by a specific carbazole derivative including a nitrogen-containing heteroaryl group.

US 2017/0125699 disclose an organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by a specific biscarbazole derivative wherein the carbazole groups are linked by a linking group L₁, and a second host compound is represented by a specific carbazole derivative including a nitrogen-containing heteroaryl group.

In the preparation/application examples in US 2017/0117488, US 2017/0170408, US 2017/0213968, US 2017/0207396 and US 2017/0125699 the first host and the second host are each evaporated from a different evaporation source.

When two materials (e.g., the first host and the second host) are respectively evaporated from different evaporation sources, the manufacturing process of the organic electroluminescence device becomes complicated. However, an organic electroluminescence device including a single material (e.g. only one of the first host and the second host) usually exhibits a worse performance, for example a shorter lifetime and/or lower efficiency. For this reason, a possibility of stably evaporating two materials (or a plurality of materials) from a single evaporation source is desired.

An object of the invention is therefore to provide a composition capable of being evaporated from a single evaporation source at a stable material ratio while maintaining a good performance of an organic electroluminescence device comprising the composition (e.g., at least one luminescent performance of driving voltage, luminous efficiency or lifetime), to provide an organic electroluminescence device material comprising the composition, to provide a composition film comprising the composition, to provide an organic electroluminescence device comprising the composition, and to provide an electronic device comprising the organic electroluminescence device.

This object is achieved by providing a composition comprising
(i) a compound of formula (1) 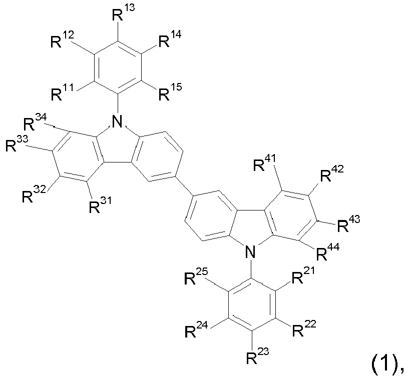 and
(ii) a compound of formula (2) 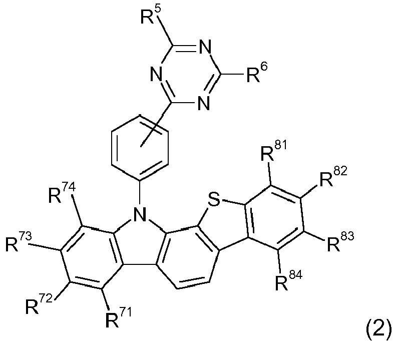 wherein
   R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ each independently represent hydrogen, CN, an unsubstituted phenyl group, a phenyl group substituted by at least one CN group, an unsubstituted biphenyl group, a biphenyl group substituted by at least one CN group, an unsubstituted dibenzofuran group, a dibenzofuran group substituted by at least one CN group, an unsubstituted dibenzothiophene group or a dibenzothiophene group substituted by at least one CN group, wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12; R⁵ represents an unsubstituted phenyl group;
   R⁶ represents an unsubstituted phenyl group, an unsubstituted biphenyl group, an unsubstituted dibenzofuran group or an unsubstituted dibenzothiophene group;
   R⁷¹, R⁷², R⁷³, R⁷⁴, R⁸¹, R⁸², R⁸³ and R⁸⁴ each independently represent hydrogen or an unsubstituted phenyl group, preferably hydrogen;
   wherein the sum of ring carbon atoms of the residues R⁵, R⁶, R⁷¹, R⁷², R⁷³, R⁷⁴, R⁸¹, R⁸², R⁸³ and R84 is exactly 18.

The object is further solved by providing an organic electroluminescence device material comprising the composition according to the present invention; by providing a composition film comprising the composition according to the present invention; by providing an organic EL device including: an anode; a cathode; and at least one organic layer provided between the anode and the cathode, in which the at least one organic layer includes the composition according to the present invention; and by providing an electronic device including the organic electroluminescence device according to the present invention.

According to the present invention a composition is provided capable of being evaporated from a single evaporation source at a stable material ratio. Organic electroluminescence devices comprising said composition show a good overall performance (e.g., at least one luminescent performance of driving voltage, luminous efficiency and lifetime).

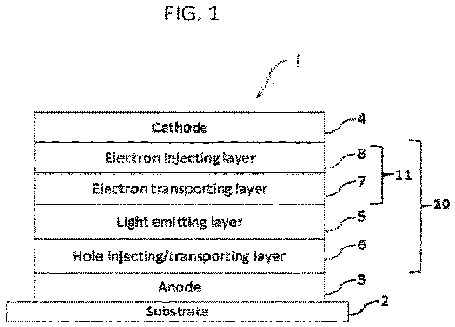

**FIG. 1** schematically shows an exemplary arrangement of an organic electroluminescence device according to the present invention.

The residues mentioned in the description of the present application generally have the following preferred meanings, if said residues are not further specified in specific embodiments mentioned:
A hydrogen atom encompasses isotopes having different numbers of neutrons, specifically, protium, deuterium and tritium.

The "sum of ring carbon atoms" means the number of carbon atoms forming the ring itself of a residue in which the atoms are bonded to form the ring (e.g., a monocyclic compound, a fused ring compound, a carbocyclic compound, and a heterocyclic compound). When the ring is substituted by a substituent, the "ring carbon atoms" do not include carbon(s) contained in the substituent. For instance, a phenyl group has 6 ring carbon atoms, a biphenyl group has 12 ring carbon atoms, a dibenzofuran group has 12 ring carbon atoms, and a dibenzothiophene group has 12 ring carbon atoms. When the phenyl group, the biphenyl group, the dibenzofuran group and/or the dibenzothiophene group is substituted by at least one CN group, the carbon atom of the CN group is not included in the number of the ring carbon atoms.

"Unsubstituted" in the description of "substituted or unsubstituted" herein means that a group is not substituted by CN but bonded with a hydrogen atom.

The substituent in the description of "substituted or unsubstituted" is according to the present application a CN group.

"Substituted by at least one CN group" means that the phenyl group, the biphenyl group, the dibenzofuran group and/or the dibenzothiophene group may be substituted by one or more CN groups, preferably by 1, 2, 3, 4 or 5 CN groups, more preferably by 1 or 2 CN groups, most preferably by 1 CN group.

Most preferably, the phenyl group, the biphenyl group, the dibenzofuran group and the dibenzothiophene group are unsubstituted.

### Composition

The composition comprises a first compound represented by formula (1) and a second compound represented by formula (2). Preferably, the composition according to the present invention consists of compounds of at least one compound of formula (1) and at least one compound of formula (2). More preferably, the composition according to the present invention consists of compounds of one compound of formula (1) and one compound of formula (2). 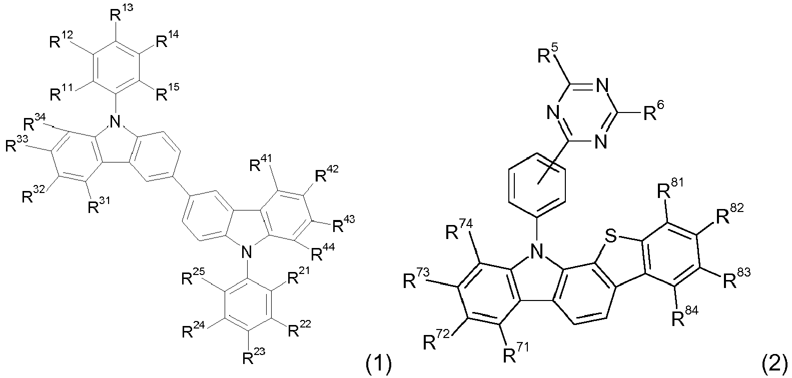 wherein the residues and indices are defined above and below.

The inventive composition may be in any form. Examples of the forms of the inventive composition include solid, powder, solution and film. When the inventive composition is solid, the composition may be pelletized.

### First Compound

The first compound is a compound of formula (1) 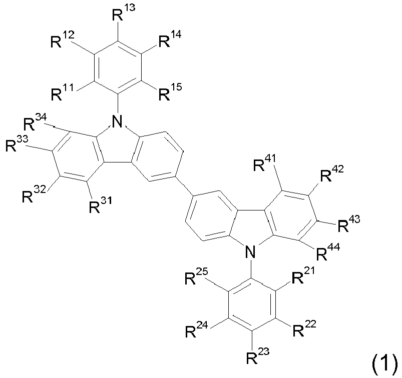 wherein
R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ each independently represent hydrogen, CN, an unsubstituted phenyl group, a phenyl group substituted by at least one, preferably one, CN group, an unsubstituted biphenyl group, a biphenyl group substituted by at least one, preferably one, CN group, an unsubstituted dibenzofuran group, a dibenzofuran group substituted by at least one, preferably one, CN group, an unsubstituted dibenzothiophene group or a dibenzothiophene group substituted by at least one, preferably one, CN group, wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

Preferably,
R¹¹, R¹², R¹³, R¹⁴, R¹⁵, R²¹, R²², R²³, R²⁴ and R²⁵ each independently represent hydrogen, CN, an unsubstituted phenyl group, a phenyl group substituted by at least one, preferably one, CN group, an unsubstituted biphenyl group, a biphenyl group substituted by at least one, preferably one, CN group, an unsubstituted dibenzofuran group, a dibenzofuran group substituted by at least one, preferably one, CN group, an unsubstituted dibenzothiophene group or a dibenzothiophene group substituted by at least one, preferably one, CN group, and R³¹, R³², R³³, R³⁴, R⁴¹, R⁴², R⁴³ and R⁴⁴ each independently represent hydrogen, CN, an unsubstituted phenyl group, or a phenyl group substituted by at least one, preferably one, CN group; wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

More preferably,
R¹¹, R¹², R¹³, R¹⁴, R¹⁵, R²¹, R²², R²³, R²⁴ and R²⁵ each independently represent hydrogen, CN, an unsubstituted phenyl group, a phenyl group substituted by at least one, preferably one, CN group, an unsubstituted biphenyl group, an unsubstituted dibenzofuran group, or an unsubstituted dibenzothiophene group; and
R³¹, R³², R³³, R³⁴, R⁴¹, R⁴², R⁴³ and R⁴⁴ each independently represent hydrogen, CN, an unsubstituted phenyl group, or a phenyl group substituted by at least one, preferably one, CN group; wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

Most preferably,
R¹¹, R¹², R¹³, R¹⁴, R¹⁵, R²¹, R²², R²³, R²⁴ and R²⁵ each independently represent hydrogen, an unsubstituted phenyl group, an unsubstituted biphenyl group or an unsubstituted dibenzofuran group; and
R³¹, R³², R³³, R³⁴, R⁴¹, R⁴², R⁴³ and R⁴⁴ each independently represent hydrogen or an unsubstituted phenyl group;
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

Further most preferably,
R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ each independently represent hydrogen or an unsubstituted phenyl group;
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

In a further most preferred embodiment,
one, two, three or four of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ have the following meanings:
R¹¹, R¹², R¹³, R¹⁴, R¹⁵, R²¹, R²², R²³, R²⁴ and R²⁵ each independently represent CN, an unsubstituted phenyl group, a phenyl group substituted by at least one, preferably one, CN, an unsubstituted biphenyl group, an unsubstituted dibenzofuran group, or an unsubstituted dibenzothiophene group, and
R³¹, R³², R³³, R³⁴, R⁴¹, R⁴², R⁴³ and R⁴⁴ each independently represent CN, an unsubstituted phenyl group, or a phenyl group substituted by at least one, preferably one, CN; and
the remaining residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ are hydrogen;
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

In a further more most preferred embodiment,
one or two of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ have the following meanings:
R¹¹, R¹², R¹³, R¹⁴, R¹⁵, R²¹, R²², R²³, R²⁴ and R²⁵ each independently represent CN, an unsubstituted phenyl group, a phenyl group substituted by at least one, preferably one, CN, an unsubstituted biphenyl group, an unsubstituted dibenzofuran group, or an unsubstituted dibenzothiophene group, and
R³¹, R³², R³³, R³⁴, R⁴¹, R⁴², R⁴³ and R⁴⁴ each independently represent CN, an unsubstituted phenyl group, or a phenyl group substituted by at least one, preferably one, CN; and
the remaining residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ are hydrogen;
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

In a further even more most preferred embodiment,
one or two of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ have the following meanings:
one of R¹¹, R¹², R¹³, R¹⁴, R¹⁵ and/or one of R²¹, R²², R²³, R²⁴ and R²⁵ each independently represent CN, an unsubstituted phenyl group, a phenyl group substituted by at least one, preferably one, CN, an unsubstituted biphenyl group, an unsubstituted dibenzofuran group, or an unsubstituted dibenzothiophene group, and
one of R³¹, R³², R³³, R³⁴ and/or one of R⁴¹, R⁴², R⁴³ and R⁴⁴ each independently represent CN, an unsubstituted phenyl group, or a phenyl group substituted by at least one, preferably one, CN; and
the remaining residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ are hydrogen;
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

Further even more most preferably,
one or two of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ have the following meanings:
one of R¹¹, R¹², R¹³, R¹⁴, R¹⁵ and/or one of R²¹, R²², R²³, R²⁴ and R²⁵ each independently represent hydrogen, an unsubstituted phenyl group, an unsubstituted biphenyl group or an unsubstituted dibenzofuran group; and
R³¹, R³², R³³, R³⁴, R⁴¹, R⁴², R⁴³ and R⁴⁴ each independently represent hydrogen or an unsubstituted phenyl group;
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

Further more most preferably,
two of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ each independently represent an unsubstituted phenyl group; and the remaining residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ are hydrogen,
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

In a further preferred embodiment, one or two of residues R¹¹, R¹², R¹³, R¹⁴ and R¹⁵ and none, one or two of the residues R²¹, R²², R²³, R²⁴ and R²⁵ each independently represent CN, an unsubstituted phenyl group, a phenyl group substituted by at least one CN group, an unsubstituted biphenyl group, a biphenyl group substituted by at least one CN group, an unsubstituted dibenzofuran group, a dibenzofuran group substituted by at least one CN group, an unsubstituted dibenzothiophene group or a dibenzothiophene group substituted by at least one CN group, preferably CN, an unsubstituted phenyl group, a phenyl group substituted by at least one, preferably one, CN group, an unsubstituted biphenyl group, an unsubstituted dibenzofuran group, or an unsubstituted dibenzothiophene group; more preferably an unsubstituted phenyl group; and the remaining residues R¹¹, R¹², R¹³, R¹⁴, R¹⁵, R²¹, R²², R²³, R²⁴ and R²⁵ are hydrogen,
wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12.

Most preferred are compounds of formula (1), wherein R³¹, R³², R³³, R³⁴, R⁴¹, R⁴², R⁴³ and R⁴⁴ are hydrogen.

The biphenyl group in the residues R¹¹, R¹², R¹³, R¹⁴, R¹⁵, R²¹, R²², R²³, R²⁴ and R²⁵ is preferably a m- or p-biphenyl group.

In the case that one or two CN groups are present in the phenyl or biphenyl residues of formula (1), said CN groups are preferably present in para or meta position of the phenyl ring to which they are bonded.

Preferred compounds of formula (1) are represented by the following formula (1a): 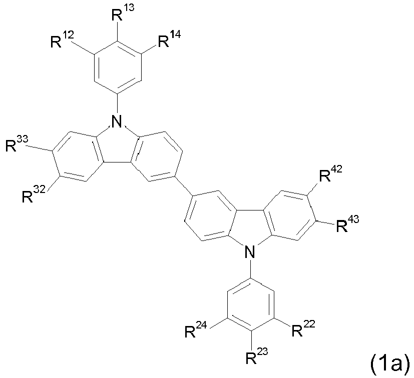 wherein
R¹², R¹³, R¹⁴, R²², R²³, R²⁴, R³², R³³, R⁴² and R⁴³ have the meanings mentioned above. More preferably, the sum of R¹², R¹³, R¹⁴, R²², R²³, R²⁴, R³², R³³, R⁴² and R⁴³ is one, two, three or four, preferably one or two.

More preferred compounds of formula (1a) are represented by the following formulae (1aa), (1ab), (1ac) and (1ad): 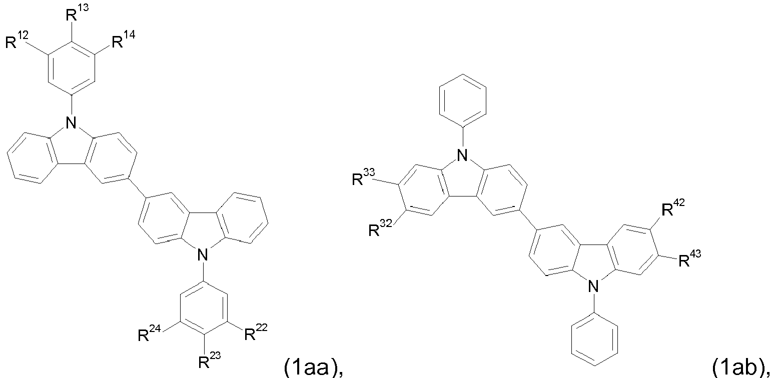 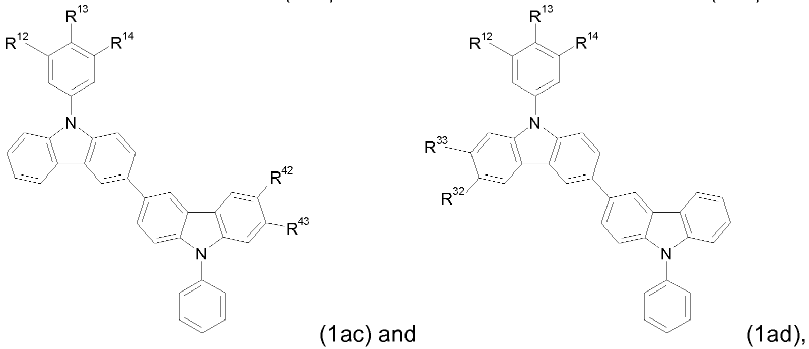 wherein
R¹², R¹³, R¹⁴, R²², R²³, R²⁴, R³², R³³, R⁴² and R⁴³ have the meanings mentioned above, wherein a compound of formula (1aa) is most preferred.

Further most preferably,
in formula (1aa): the sum of the number of residues R¹², R¹³, R¹⁴ and R²², R²³, R²⁴ is one or two;
in formula (1ab): the sum of the number of residues R³², R³³, R⁴² and R⁴³ is one or two, preferably two;
in formula (1ac): the sum of the number of residues R¹², R¹³, R¹⁴, R⁴² and R⁴³ is one or two, preferably two;
in formula (1ad): the sum of the number of residues R¹², R¹³, R¹⁴, R³² and R³³ is one or two, preferably two.

Examples of the compounds of formula (1) are given below. It should be noted that the compounds of formula (1) are by no means limited to the examples below.

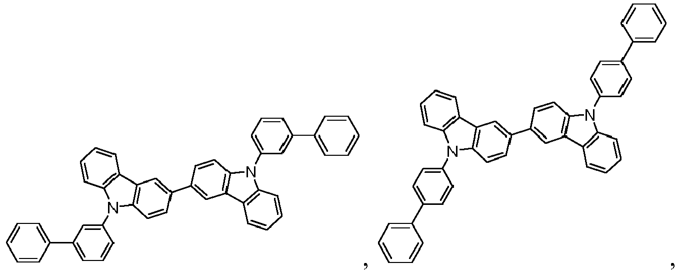

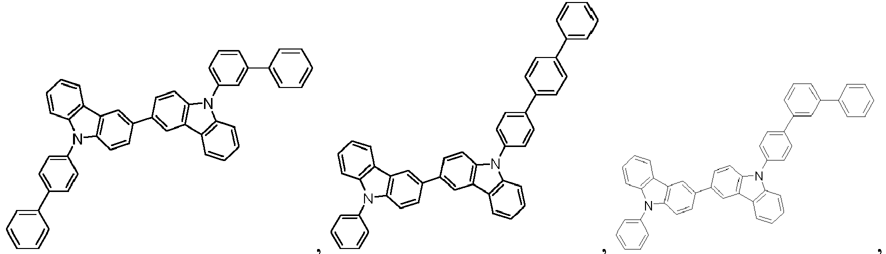

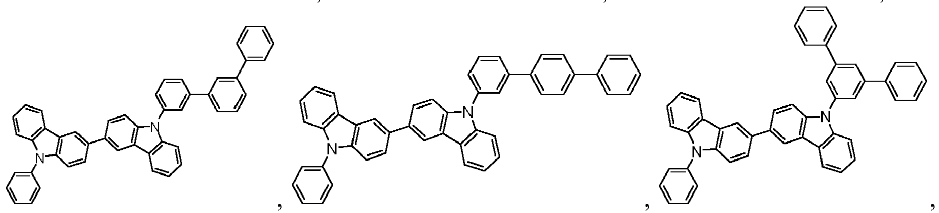

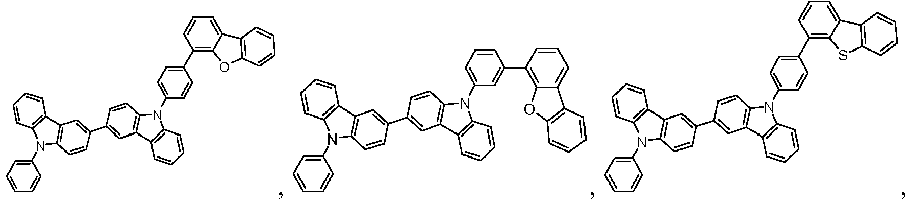

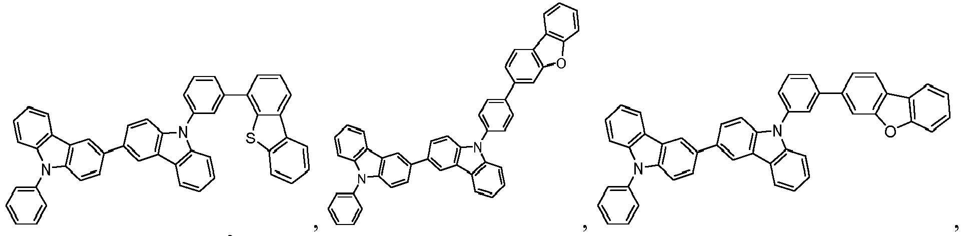

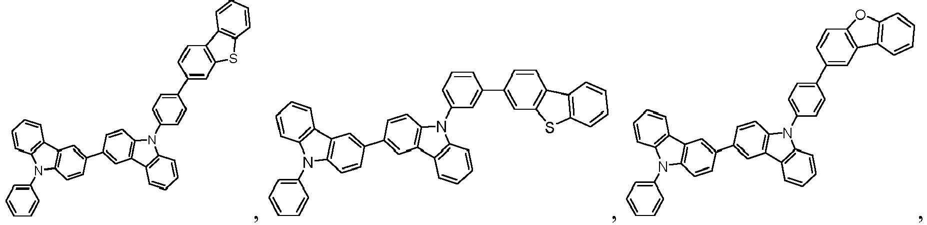

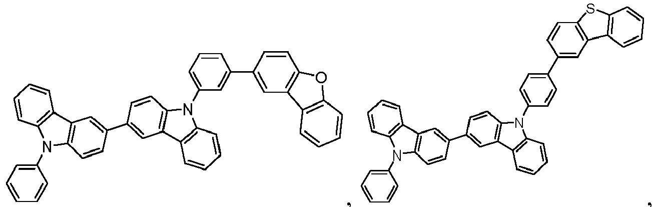

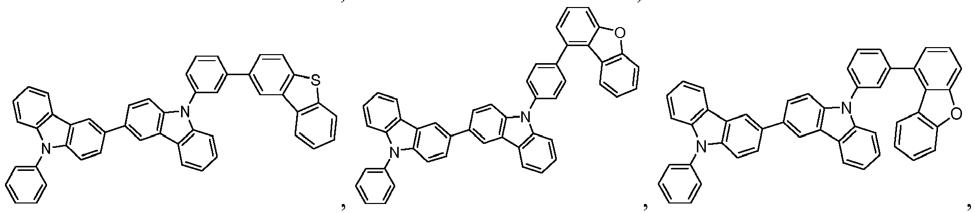

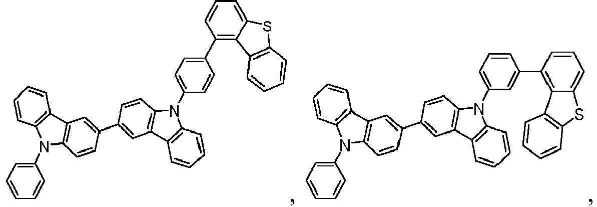

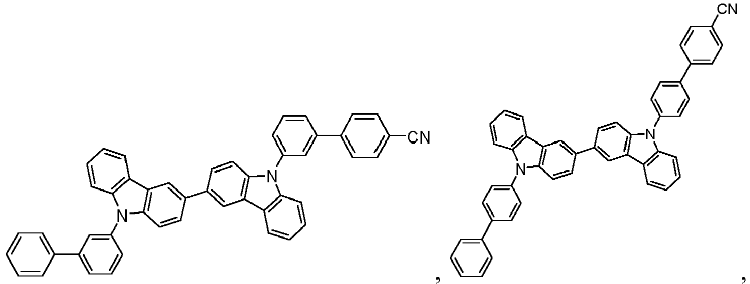

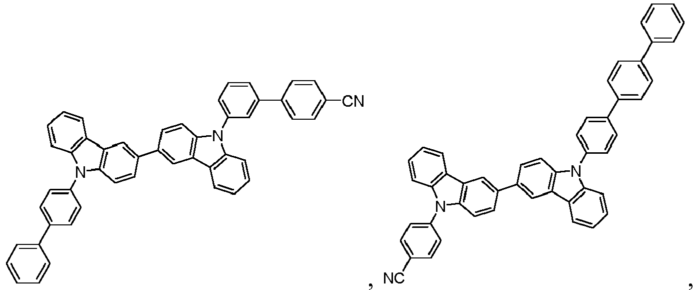

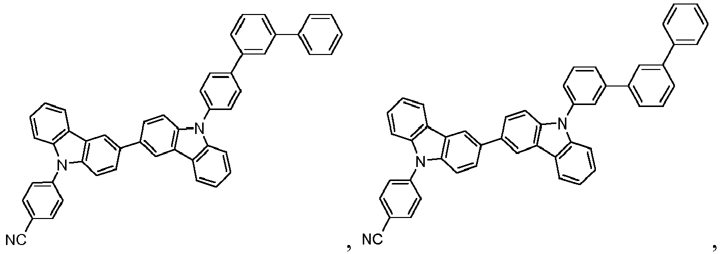

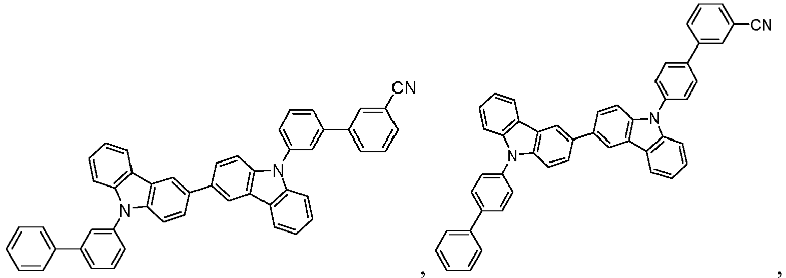

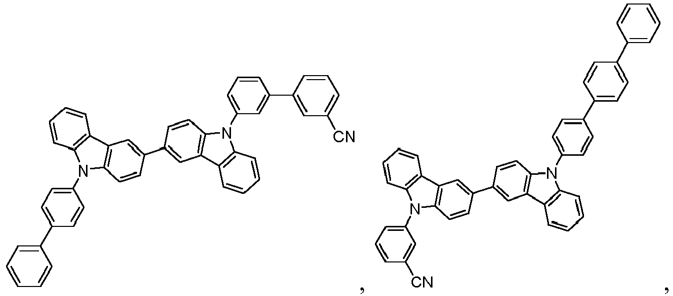

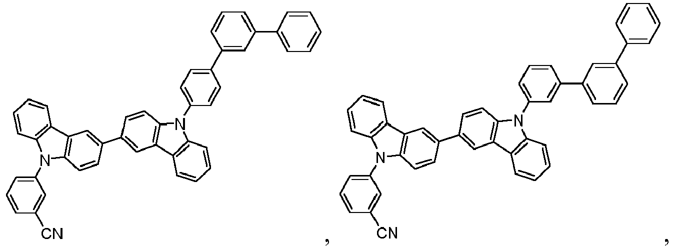

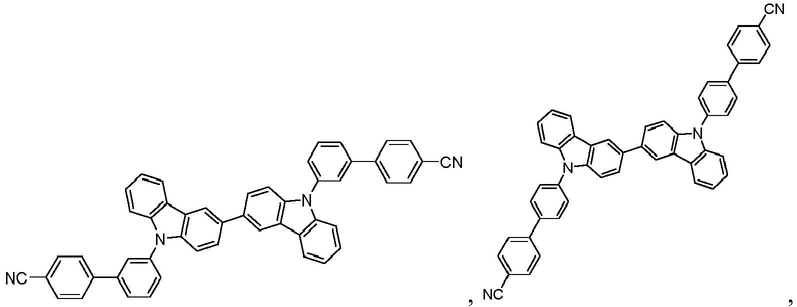

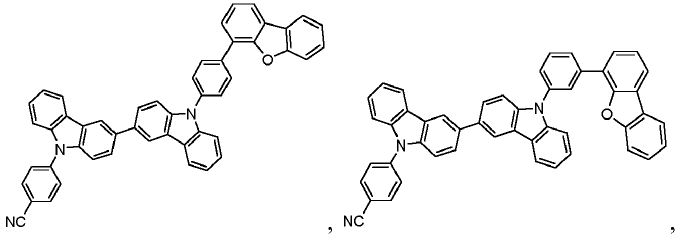

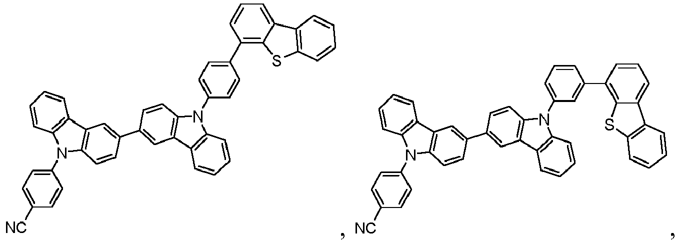

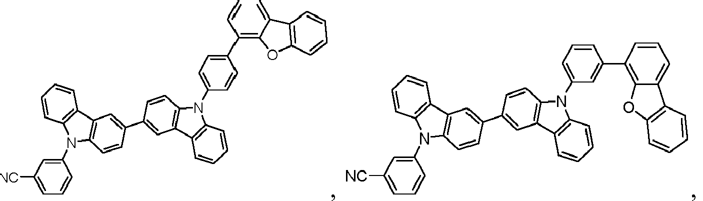

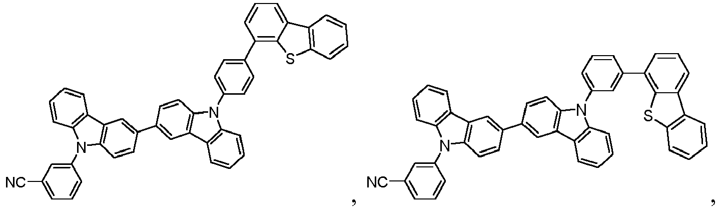

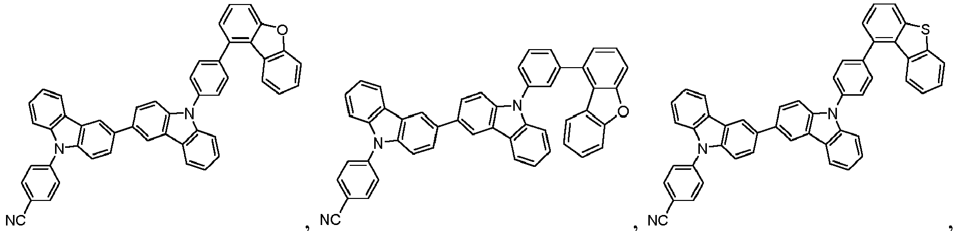

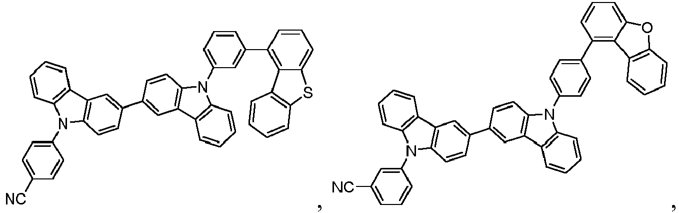

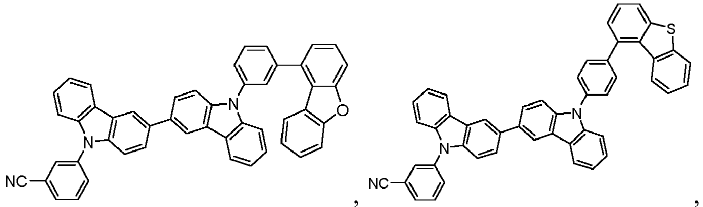

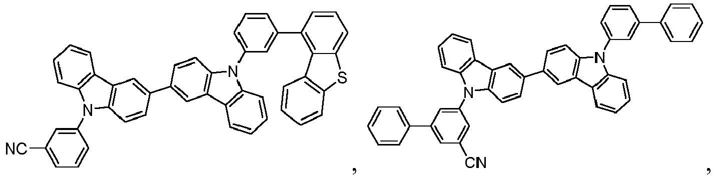

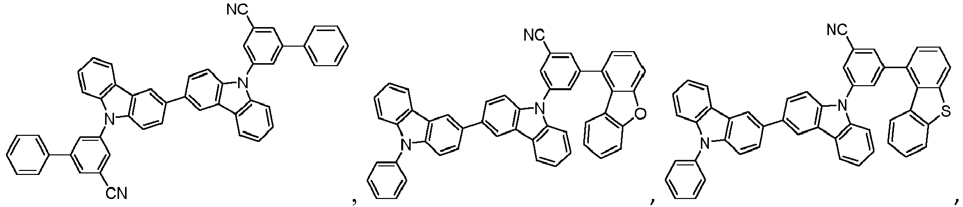

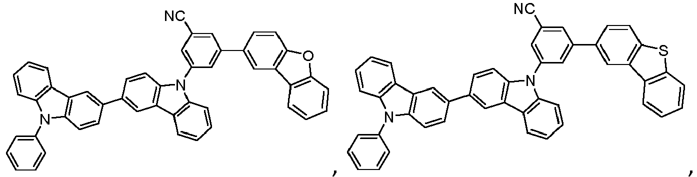

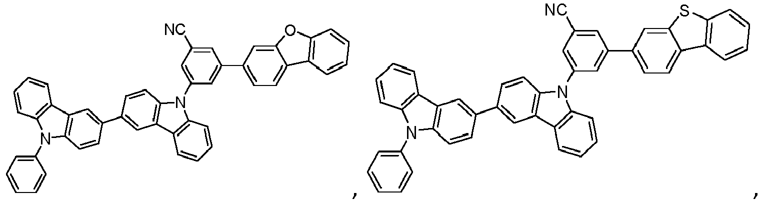

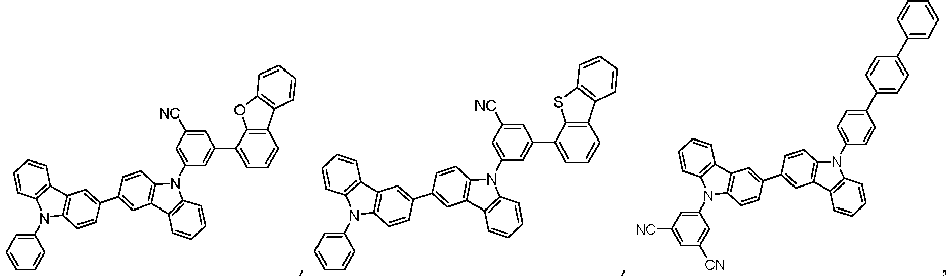

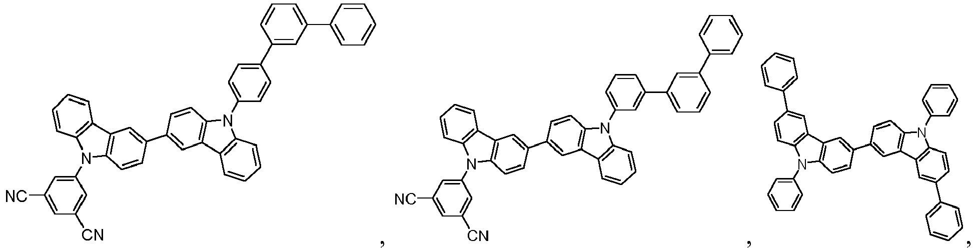

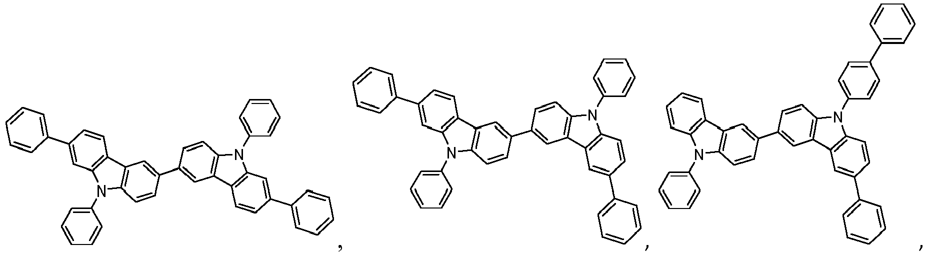

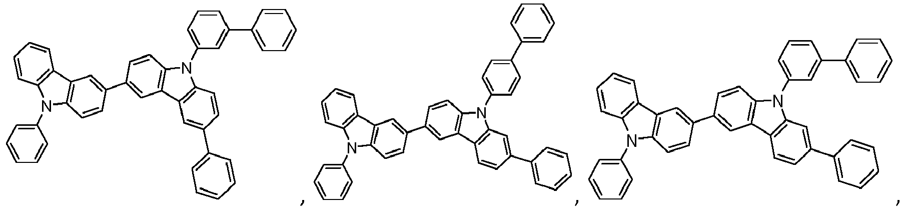

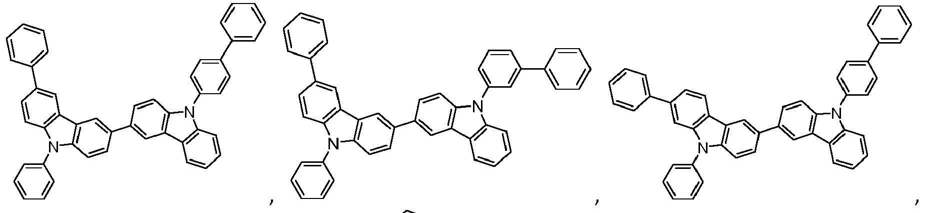

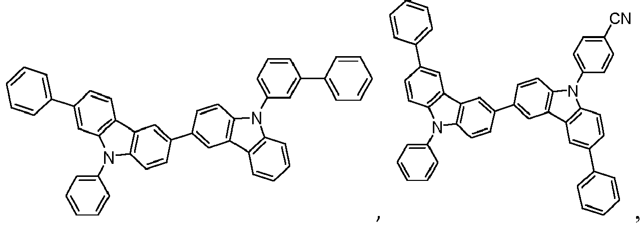

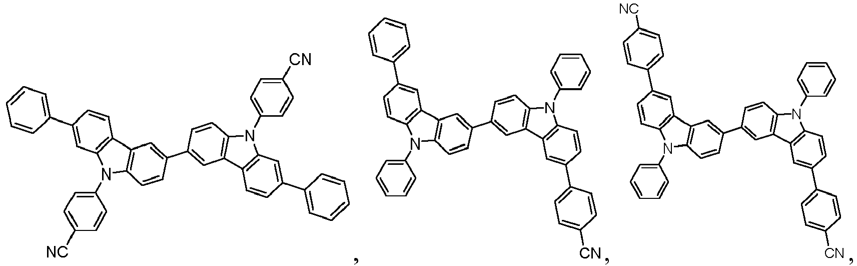

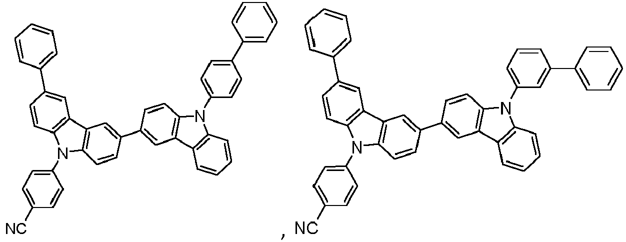

The compounds of formula (1) can be manufactured by known methods or in analogy to known methods as for example described in EP 2 674 429, US 20130313536, WO 2015/190718, KR 10-2015-0141047 and KR-10-2016-0111351.

### Second Compound

The second compound is a compound of formula (2) 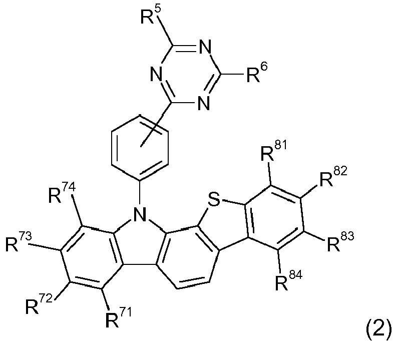 wherein
R⁵ represents an unsubstituted phenyl group, preferably an unsubstituted phenyl group;
R⁶ represents an unsubstituted phenyl group, an unsubstituted biphenyl group, an unsubstituted dibenzofuran group or an unsubstituted dibenzothiophene group, preferably an unsubstituted phenyl group, an unsubstituted biphenyl group, an unsubstituted dibenzofuran group or an unsubstituted dibenzothiophene group, more preferably an unsubstituted biphenyl group, an unsubstituted dibenzofuran group or an unsubstituted dibenzothiophene group;
R⁷¹, R⁷², R⁷³, R⁷⁴, R⁸¹, R⁸², R⁸³ and R⁸⁴ each independently represent hydrogen or an unsubstituted phenyl group, preferably hydrogen;
wherein the sum of ring carbon atoms of the residues R⁵, R⁶, R⁷¹, R⁷², R⁷³, R⁷⁴, R⁸¹, R⁸², R⁸³ and R84 is exactly 18.

Preferably, R⁷¹, R⁷², R⁷³, R⁷⁴, R⁸¹, R⁸², R⁸³ and R⁸⁴ are hydrogen. In this case, R⁵ represents an unsubstituted phenyl group and R⁶ represents an unsubstituted biphenyl group, an unsubstituted dibenzofuran group or an unsubstituted dibenzothiophene group.

Preferred compounds of formula (2) are represented by formulae (2a), (2b) and (2c) 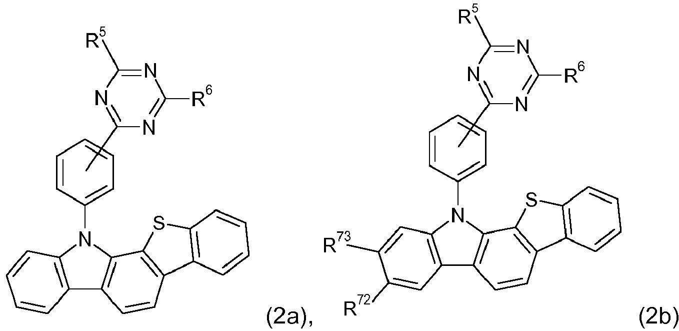 and 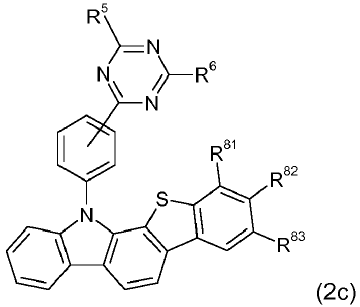 wherein the residues R⁵, R⁶, R⁷², R⁷³, R⁸¹, R⁸² and R⁸³ are defined above, preferably the compounds of formula (2) are represented by formula (2a).

In formula (2a):
R⁵ represents an unsubstituted phenyl group; and
R⁶ represents an unsubstituted biphenyl group, an unsubstituted dibenzofuran group or an unsubstituted dibenzothiophene group.

Preferably, in formula (2b):
R⁵ represents an unsubstituted phenyl group; and
R⁶ represents an unsubstituted phenyl group; and
one of R⁷² and R⁷³ represents an unsubstituted phenyl group, and the other of R⁷² and R⁷³ represents hydrogen.

Preferably, in formula (2c):
R⁵ represents an unsubstituted phenyl group; and
R⁶ represents an unsubstituted phenyl group; and
one of R⁸¹, R⁸² and R⁸³ represents an unsubstituted phenyl group, and the other two of R⁸¹, R⁸² and R⁸³ represent hydrogen.

Examples of the compounds of formula (2) are given below. It should be noted that the compounds of formula (2) are by no means limited to the examples below.

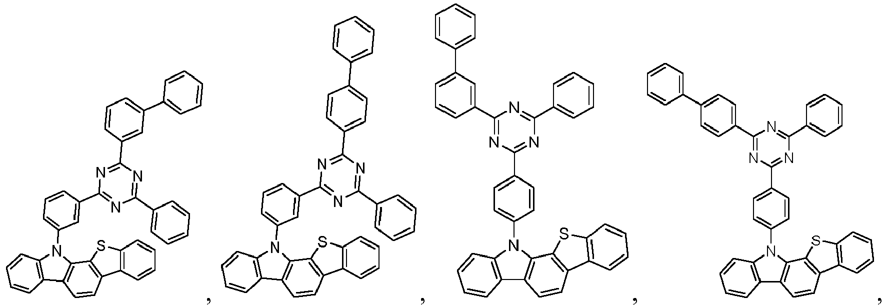

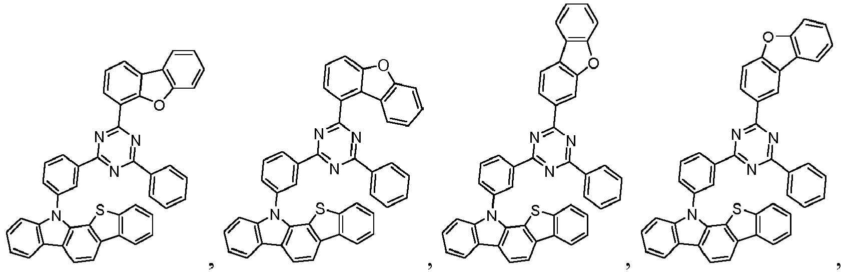

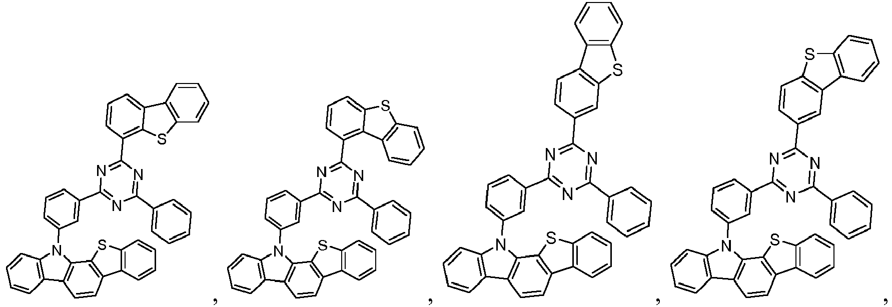

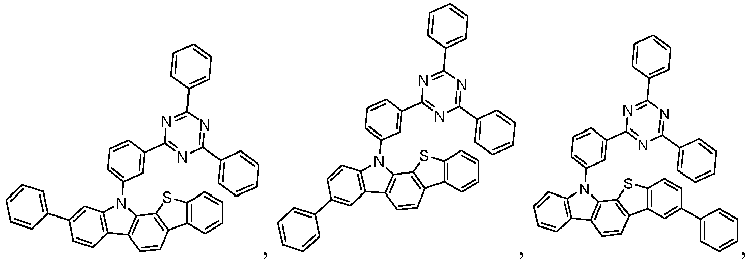

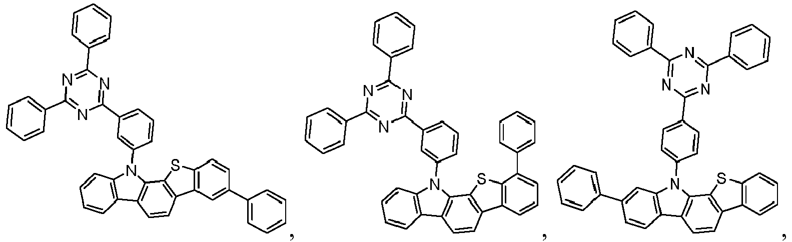

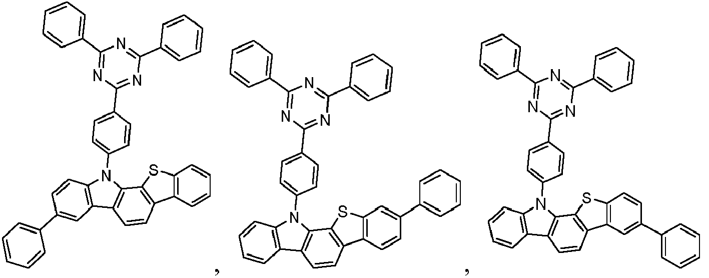

The compounds of formula (2) can be manufactured by known methods or in analogy to known methods as for example described in the art, for example in KR2016/125022.

The 12*H*-[1]benzothieno[2,3-*a*]carbazole can be prepared according to e.g. KR2016/125022. Further derivatisation of the NH group of the building block can be achieved using known procedures such as Ullmann of Buchwald reactions.

The composition according to the present invention can be evaporated from a single evaporation source at a stable material ratio. The overall performance of the organic electroluminescence device comprising said composition is good (e.g., at least one luminescent performance of a driving voltage, a luminous efficiency and a lifetime). A stable material ratio is in the meaning of the present application generally a material ratio with a deviation between the initial ratio (mass ratio) (at the evaporation initial stage) and the residual ratio (at the evaporation terminal stage) from a single evaporation source of 0 to 10%, preferably of 0 to 5%, more preferably of 0 to 3%.

The residual ratio is determined as follows: A crucible filled with the first compound (compound of formula (1)) and the second compound (compound of formula (2)) is set in a vacuum evaporator and the mixture is evaporated onto a glass substrate until 80% of the original content in the crucible is deposited on the glass substrate. The residue in the crucible is taken out of the evaporator and is dissolved in tetrahydrofuran. Based on a peak area value of each of components detected by HPLC, the ratio (residual ratio) between the two compounds is calculated.

### Compounding Ratio of Composition

The compounding ratio of the first compound (compound of formula (1)) and the second compound (at least one, preferably one, compound of formula (2)) is not particularly limited. The compounding ratio of the first compound and the second compound only needs to be appropriately determined depending on desired effects of the composition. The initial compounding ratio (mass ratio) represented by the first compound: the second compound is usually in a range from 1:99 to 99:1, preferably in a range from 10:90 to 90:10, more preferably in a range from 20:80 to 80:20, most preferably in a range from 30:70 to 70:30.

### Organic Electroluminescence Device Material

In a further embodiment, the present invention relates to an organic electroluminescence device material comprising the composition according to the present invention. Specifically, the organic electroluminescence device material according to the present invention includes the first compound (compound of formula (1)) and the second compound (at least one, preferably one, compound of formula (2)).

The organic electroluminescence device material according to the present invention may further comprise one or more additional compounds. When the organic electroluminescence device material according to the present invention further comprises at least one additional compound, the additional compound may be solid or liquid.

### Composition Film

In a further embodiment, the present invention relates to a composition film comprising the composition according to the present invention. Specifically, a film containing the composition according to the present invention (hereinafter, also referred to as a composition film) means a film containing the first compound (compound of formula (1)) and the second compound (at least one, preferably one, compound of formula (2)).

The composition film according to the present invention may further comprise one or more additional compounds.

A forming method of the composition film according to the present invention is not particularly limited unless otherwise specified herein. Known methods such as a dry film-forming method and a wet film-forming method are usable as a forming method of the composition film. Examples of the dry film-forming method include vacuum evaporation, sputtering, plasma deposition and ion plating. Examples of the wet film-forming include spin coating, dipping, flow coating and ink-jet.

### Device Arrangement of Organic Electroluminescence Device (Organic EL Device)

In a further embodiment, the present invention relates to an organic EL device comprising an anode, a cathode, and an organic layer between the anode and the cathode, wherein the organic layer comprises the composition according to the present invention, i.e. comprising the first compound (compound of formula (1)) and the second compound (at least one, preferably one, compound of formula (2)).

The organic layer includes at least one layer formed from one or more organic compounds. Alternatively, the organic layer is a laminate of a plurality of layers formed from one or more organic compounds. The organic layers may further comprise one or more inorganic compounds. The organic EL device according to the present invention comprises an emitting layer. Accordingly, the organic layer may be a single emitting layer, or may further include one or more layers usable for the organic EL device. The layer(s) usable for the organic EL device are not particularly limited but are at least one layer selected from the group consisting of a hole injecting layer, a hole transporting layer, an electron injecting layer, an electron transporting layer, and a blocking layer.

In the present invention, it is preferably that the organic layer of the organic EL device comprises a plurality of organic layers and one of the plurality of organic layers comprises the composition according to the present invention.

More preferably the organic layer of the organic EL device comprises an emitting layer, and the emitting layer comprises the composition according to the present invention.

Since the composition according to the present invention is used in at least one of the layers in the organic EL device of the present invention, a high performance of the organic EL device (e.g., at least one luminescent performance of a driving voltage, a luminous efficiency and a lifetime) is obtainable. Further, when the composition of the present invention is formed into a film using the method of the present invention (e.g., vacuum evaporation), the material ratio between first compound (compound of formula (1)) and the second compound (at least one, preferably one, compound of formula (2)) in the organic layer is stable during an evaporation time from an evaporation initial stage to an evaporation terminal stage. As a result, the organic electroluminescence device can maintain a high and stable luminescent performance irrespective of the evaporation time.

Preferably, the composition according to the present invention is present in the emitting layer of the organic EL device. The emitting layer either comprises the composition according to the present invention or consists of the composition according to the present invention. The composition of the present invention may be used in the emitting layer without being used in a further layer of the organic EL device, or the composition of the present invention may be used in the emitting layer with being used in a further layer, preferably in the electron transporting layer, of the organic EL device.

Therefore, the present invention also relates to an emitting layer comprising the composition of the present invention.

Preferably, the organic EL device further comprises a hole transporting layer between the anode and the emitting layer.

Further preferably, the organic EL device further comprises an electron transporting layer between the cathode and the emitting layer.

In a further embodiment, the composition of the present invention is preferably used in the electron transporting layer of the organic EL device. The composition of the present invention may be used in the electron transporting layer without being used in a further layer of the organic EL device, or the composition of the present invention may be used in the electron transporting layer with being used in a further layer, preferably in the emitting layer, of the organic EL device.

Most preferably, the composition of the present invention is used in the emitting layer. In other words, the emitting layer comprises the first compound (compound of formula (1)) and the second compound (at least one, preferably one, compound of formula (2)). Further most preferably, the composition of the present invention is used in the emitting layer without being used in a further layer of the organic EL device.

Typical device arrangements of the organic EL device include the following arrangements (a) to (f) and the like:
(a) anode / emitting layer/ cathode;
(b) anode / hole injecting transporting layer / emitting layer / cathode;
(c) anode / emitting layer / electron injecting transporting layer / cathode;
(d) anode / hole injecting transporting layer / emitting layer / electron injecting transporting layer / cathode.
(e) anode / hole injecting transporting layer / emitting layer / blocking layer / electron injecting transporting layer / cathode; and
(f) anode / hole injecting·transporting layer / blocking layer / emitting layer / blocking layer / electron injecting·transporting layer / cathode.

While the arrangement (d) is preferably used among the above device arrangements, the arrangement of the invention is not limited to the above. It should be noted that the above-described "emitting layer" is an organic layer having an emitting function. The above-described "hole injecting·transporting layer" means at least one of a hole injecting layer and a hole transporting layer." The above-described "electron injecting·transporting layer" means at least one of an electron injecting layer and an electron transporting layer." When the organic EL device includes the hole injecting layer and the hole transporting layer, the hole injecting layer is preferably provided between the hole transporting layer and the anode. When the organic EL device includes the electron injecting layer and the electron transporting layer, the electron injecting layer is preferably provided between the electron transporting layer and the cathode. Each of the hole injecting layer, the hole transporting layer, the electron transporting layer, and the electron injecting layer may consist of a single layer or a plurality of layers.

FIG. 1 shows a schematic configuration of one example of the organic EL device of the invention. The organic EL device 1 comprises a substrate 2, an anode 3, a cathode 4 and an emitting unit 10 provided between the anode 3 and the cathode 4. The emitting unit 10 comprises an emitting layer 5 preferably comprising a host material and a dopant. A hole injecting and transporting layer 6 or the like may be provided between the emitting layer 5 and the anode 3 and an electron-injecting layer 8 and an electron transporting layer 7 or the like (electron injecting and transporting unit 11) may be provided between the emitting layer 5 and the cathode 4. An electron-barrier layer may be provided on the anode 3 side of the emitting layer 5 and a hole-barrier layer may be provided on the cathode 4 side of the emitting layer 5. Due to such configuration, electrons or holes can be confined in the emitting layer 5, whereby possibility of generation of excitons in the emitting layer 5 can be improved.

The emitting layer 5 of the organic EL device 1 preferably comprises the composition of the present invention. In other words, the emitting layer 5 comprises the first compound (compound of formula (1)) and the second compound (at least one, preferably one, compound of formula (2)).

The organic EL device of the present invention is preferably driven at a low voltage since the first compound and the second compound are used in combination in the organic layer. In order to drive the organic EL device at a low voltage, the first compound and the second compound are preferably contained in a single emitting layer.

### Layer Formation Method(s)

A forming method of each layer of the organic EL device according to the present invention is not particularly limited unless otherwise specified herein. Known methods such as a dry film-forming method and a wet film-forming method are usable as the forming method of the layers. Examples of the dry film-forming include vacuum evaporation, sputtering, plasma deposition and ion plating. Examples of the wet film-forming include spin coating, dipping, flow coating and ink-jet.

### Film Thickness

A film thickness of each layer in the organic EL device according to the present invention is not limited. The film thickness of each layer needs to be set at an appropriate thickness. When the film thickness is too thick, a large applied voltage may be required for obtaining emission at a certain level, which may deteriorate efficiency. When the film thickness is too thin, pin holes and the like may generate, which may cause an insufficient luminescence intensity even when an electric field is applied The film thickness is typically preferably in a range from 5 nm to 10 µm, more preferably in a range of 10 nm to 0.2 µm.

Materials and the like of components of the organic EL device will be described below. According to the present invention, the composition of the present invention is present in the organic layer of the organic EL device. Preferably, the composition of the present invention is present in the emitting layer, i.e. the emitting layer comprises the first compound (compound of formula (1)) and the second compound (at least one, preferably one, compound of formula (2)). It is also possible that the composition of the present invention is present in more than one layer of the organic layer in the organic EL device.

### Substrate

A substrate is used as a support for the organic EL device. For instance, glass, quartz, plastics and the like are usable as the substrate. A flexible substrate is also usable. The flexible substrate is a bendable substrate, which is exemplified by a plastic substrate formed from polycarbonate and polyvinyl chloride.

### Anode

Metal having a large work function (specifically, 4.0 eV or more), an alloy, an electrically conductive compound and a mixture thereof are preferably usable as the anode formed on the substrate. Specific examples of the material for the anode include indium tin oxide (ITO), indium tin oxide containing silicon or silicon oxide, indium zinc oxide, tungsten oxide, indium oxide containing zinc oxide, and graphene. In addition, the examples of the material for the anode further include nitrides of gold (Au), platinum (Pt), or metal materials (e.g., titanium nitride).

### Hole Injecting Layer

The hole injecting layer is a layer for efficiently injecting holes from the anode into the organic layer. Examples of a substance used for the hole injecting layer include molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, aromatic amine compound, acceptor compound, and high-molecular compound (e.g., oligomer, den-drimer, and polymer).

Among the above examples, the substance used for the hole injecting layer is preferably an aromatic amine compound or an acceptor compound, more preferably an acceptor compound. Preferred examples of the acceptor compound include a heterocyclic compound substituted by an electron-withdrawing group, a quinone compound substituted by an electron-withdrawing group, an aryl borane compound, and a heteroaryl borane compound. Among the examples, hexacyanohexaazatriphenylene, F₄TCNQ (2,3,5,6-tetrafluoro-7,7,8,8-tetracyanoquinodimethane), or 1,2,3-tris[(cyano)(4-cyano-2,3,5,6-tetrafluorophenyl)methylene]cyclopropane is preferably used.

It is also preferable that a layer containing the acceptor compound further contains a matrix material. Organic EL device materials are widely usable as a matrix material. The matrix material used in combination with the acceptor compound is preferably a donor compound, more preferably an aromatic amine compound.

### Hole Transporting Layer

The hole transporting layer is a layer containing a highly hole-transporting substance. An aromatic amine compound, carbazole compound, anthracene compound and the like are usable for the hole transporting layer. Moreover, a high-molecular compound such as poly(N-vinylcarbazole) (abbreviation: PVK) and poly(4-vinyltriphenylamine) (abbreviation: PVTPA) is also usable for the hole transporting layer. However, any substance having a hole transporting performance higher than an electron transporting performance may be used in addition to the above substances. The layer including the highly hole-transporting substance may be provided in the form of a single layer or a laminate of two or more layers containing the above substance. A hole transporting material is preferably a compound represented by a formula (H) below.

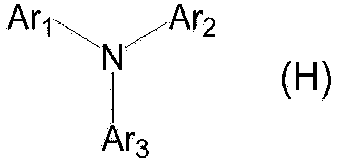

In the formula (H), Ar₁ to Ar₃ each independently represent a substituted or unsubstituted aryl group having 6 to 50 ring carbon atoms, a substituted or unsubstituted heterocyclic group having 3 to 50 ring atoms, or a group formed by combining the substituted or unsubstituted aryl group with the substituted or unsubstituted heterocyclic group. The aryl group is preferably a substituent such as a phenyl group, biphenyl group, terphenyl group, fluorenyl group, spirobifluorenyl group, indenofluorenyl group, naphthyl group, phenanthryl group, anthryl group, and triphenylenyl group. The heterocyclic group is preferably a dibenzofuranyl group, dibenzothiophenyl group, carbazolyl group and the like. The group formed by combining the aryl group with the heterocyclic group is preferably a dibenzofuran-substituted aryl group, dibenzothiophene-substituted aryl group, and carbazole-substituted aryl group. The above substituents may have an additional substituent, preferred examples of which are described below.

The compound represented by the formula (H) is preferably in a form of a compound in which at least one of Ar₁ to Ar₃ is further substituted by an aryl amino group, and is also preferably a diamine compound, a triamine compound, or a tetramine compound. Preferred examples of the diamine compound include a tetraaryl-substituted benzidine compound, and TPTE (4,4'-bis[N-phenyl-N-[4'-diphenylamino-1,1'-biphenyl-4-yl]amino]-1,1'-biphenyl).

The hole transporting material used for a layer adjacent to a phosphorescent layer preferably has a high triplet level. Each of Ar₁ to Ar₃ in the formula (H) is preferably a substituent such as a fluorenyl group, spirofluorenyl group, phenyl group, biphenyl group, phenanthryl group, triphenylenyl group, dibenzofuranyl group, and dibenzothiophenyl group, and a group formed by combining the substituents.

### Emitting Layer

The emitting layer is a layer containing a highly emittable substance and can be formed of various materials. The emitting layer typically contains a luminescent material (a dopant material). Preferably, the emitting layer contains a highly emittable luminescent material (a dopant material) and a host material for promoting an efficient emission of the luminescent material. For instance, a fluorescent compound emitting fluorescence and/or a phosphorescent compound emitting phosphorescence are usable as the highly emittable substance. The fluorescent compound is a compound capable of emitting in a singlet state. The phosphorescent compound is a compound capable of emitting in a triplet state. The emitting layer containing the fluorescent compound is referred to as a fluorescent layer. The emitting layer containing the phosphorescent compound is referred to as a phosphorescent layer.

The fluorescent compound is widely usable as a dopant material of the fluorescent layer.

The fluorescent compound preferably emits a red, green or blue fluorescence. Suitable fluorescent emitters emitting a red, green or blue fluorescence are known to a person skilled in the art.

The fluorescent compound preferably emits fluorescence having a main peak wavelength (occasionally referred to as an emission peak wavelength) of 430 to 780 nm (blue, green, red). The main peak wavelength means a peak wavelength of luminescence spectrum exhibiting a maximum luminous intensity among luminous spectra measured using a toluene solution where the second compound is dissolved at a concentration from 10⁻⁶ mol/l to 10⁻⁵ mol/l.

Preferred examples of the dopant material of the fluorescent layer include a fused polycyclic aromatic compound, styrylamine compound, fused cyclic amine compound, boron-containing compound, pyrrole compound, indole compound, and carbazole compound. More preferably, the dopant material of the fluorescent layer is a fused cyclic amine compound and a boron-containing compound. Examples of the fused cyclic amine compound include a diaminopyrene compound, diaminochrysene compound, diaminoanthracene compound, diaminofluorene compound, and diaminofluorene compound to which one or more benzofuro skeletons are fused. Examples of the boron-containing compound include a pyrromethene compound and a triphenyl borane compound. Herein, a certain compound refers to a compound having a skeleton of the certain compound as a partial structure, and the compound further encompasses a compound having the skeleton and an additional ring to form a fused ring and a compound having the skeleton, substituents of which form a ring. For instance, a fused polycyclic aromatic compound refers to a compound having a fused polycyclic aromatic skeleton as a partial structure, and further encompasses a compound having the fused polycyclic aromatic skeleton and an additional ring to form a fused ring and a compound having the fused polycyclic aromatic skeleton, substituents of which form a ring.

A general fluorescent material is usable as the host material used in the fluorescent layer. The host material used in the fluorescent layer is preferably a compound having the fused polycyclic aromatic compound as a main skeleton, particularly preferably an anthracene compound, pyrene compound, chrysene compound, naphthacene compound and the like. A particularly suitable host as a blue host material (i.e., a host material usable in combination with a blue fluorescent dopant material) and a green host material (a host material usable in combination with a green fluorescent dopant material) is an anthracene compound represented by a formula (X) below.

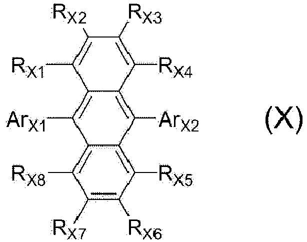

In the formula (X), Ar_{X1} and Ar_{X2} each independently represent a substituted or unsubstituted aryl group having 6 to 50 ring carbon atoms, or a substituted or unsubstituted heterocyclic group having 3 to 50 ring atoms. Preferably, Ar_{X1} and Ar_{X2} each independently represent a substituted or unsubstituted aryl group having 6 to 30 ring carbon atoms, or a heterocyclic group having 5 to 30 ring atoms.

Preferably, the emitting layer comprises a phosphorescent material as the luminescent material. A phosphorescent material (the dopant material) preferably emits a red, green or blue phosphorescence. Suitable phosphorescent emitters emitting a red, green or blue phosphorescence are known to a person skilled in the art.

The phosphorescent material preferably emits phosphorescence having a main peak wavelength (occasionally referred to as an emission peak wavelength) of 430 to 780 nm (blue, green, red). The main peak wavelength means a peak wavelength of luminescence spectrum exhibiting a maximum luminous intensity among luminous spectra measured using a toluene solution where the second compound is dissolved at a concentration from 10⁻⁶ mol/l to 10⁻⁵ mol/l.

A phosphorescent material (the dopant material) usable for the phosphorescent layer is exemplified by a metal complex such as an iridium complex, osmium complex, and platinum complex.

More preferably, the phosphorescent material is an ortho-metalated complex of a metal atom selected from the group consisting of iridium (Ir), osmium (Os) and platinum (Pt), which is preferably a complex represented by a formula (α) below.

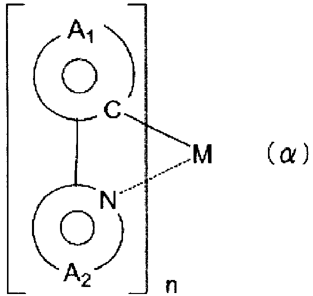

In the formula (α), M represents at least one metal selected from the group consisting of osmium, iridium, and platinum, and n represents a valence of the selected metal.
A ring A₁ represents a substituted or unsubstituted aryl group having 6 to 24 ring carbon atoms or a substituted or unsubstituted heteroaryl group having 5 to 30 ring atoms. A ring A₂ represents a substituted or unsubstituted heteroaryl group having 5 to 30 ring atoms and containing nitrogen as an atom forming a hetero ring.
The aryl group having 6 to 24 ring carbon atoms in the ring A₁ of the formula (α) is exemplified by the general definition of the aryl group mentioned above.
The heteroaryl group ring having 5 to 30 ring atoms in the rings A₁ and A₂ of the formula (α) is exemplified by the general definition of the heteroaryl group mentioned above.
The rings A₁ and A₂ of the formula (α) are unsubstituted or substituted by one or more substituents each independently selected from the group consisting of a halogen atom, a substituted or unsubstituted alkyl group having 1 to 25 carbon atoms, a substituted or unsubstituted cycloalkyl group having 3 to 25 ring carbon atoms, a substituted or unsubstituted aryl group having 6 to 18 ring carbon atoms, a substituted or unsubstituted heterocyclic group having 6 to 18 ring atoms, a silyl group substituted by at least one group selected from the group consisting of an alkyl group having 1 to 25 carbon atoms and an aryl group having 6 to 18 ring carbon atoms, or a cyano group.

Further preferably, the complex represented by the formula (α) is a complex represented by a formula (T) or (U) below.

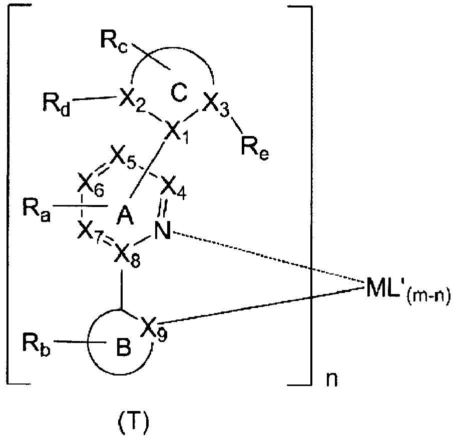

In the formula (T), M represents metal, and rings B and C each independently represent an aryl group or heteroaryl group having 5 or 6 ring atoms.

Ring A-Ring B represent a bonding pair of a heteroaryl group and an aryl or heteroaryl group and are coordinated with the metal M through a nitrogen atom of the ring A and an sp² composite atom of the ring B.

Ring A-Ring C represent a bonding pair of a heteroaryl group and an aryl or heteroaryl group.

Rₐ, R_{b} and R_{c} each independently represent one selected from the group consisting of a hydrogen atom, a substituted or unsubstituted alkyl group having 1 to 25 carbon atoms, a substituted or unsubstituted alkoxy group having 1 to 25 carbon atoms, a substituted or unsubstituted amino group, a substituted or unsubstituted alkenyl group having 2 to 25 carbon atoms, a substituted or unsubstituted alkynyl group having 2 to 25 carbon atoms, a substituted or unsubstituted aralkyl group having 7 to 50 carbon atoms, a substituted or unsubstituted aryl group having 6 to 24 ring carbon atoms, and a substituted or unsubstituted heteroaryl group having 5 to 30 ring atoms.

The number of Rₐ, R_{b} and R_{c} each independently ranges from 1 to 4.

X₁ to Xg each independently represent a carbon atom or a nitrogen atom.

R_{d} and Rₑ each independently represent one selected from the group consisting of a hydrogen atom, a substituted or unsubstituted alkyl group having 1 to 25 carbon atoms, a substituted or unsubstituted alkoxy group having 1 to 25 carbon atoms, a substituted or unsubstituted amino group, a substituted or unsubstituted alkenyl group having 2 to 25 carbon atoms, a substituted or unsubstituted alkynyl group having 2 to 25 carbon atoms, a substituted or unsubstituted aralkyl group having 7 to 50 carbon atoms, a substituted or unsubstituted aryl group having 6 to 24 ring carbon atoms, and a substituted or unsubstituted heteroaryl group having 5 to 30 ring atoms. At least one of R_{c}, R_{d} and Rₑ bonded to the ring C is not a hydrogen atom.

m represents an oxidized state of the metal M. n is 1 or more. L' represents a mono-anionic bidentate ligand.

In the formula (T), M is exemplified by osmium, iridium and platinum, among which iridium is preferable.

The aryl group having 5 or 6 ring atoms represented by the ring B and the ring C is exemplified by the general definition of the aryl group mentioned above.

The heteroaryl group having 5 or 6 ring atoms represented by the ring B and the ring C is exemplified by the general definition of the heteroaryl group mentioned above.

The substituted or unsubstituted alkyl group having 1 to 25 carbon atoms, substituted or unsubstituted alkoxy group having 1 to 25 carbon atoms, substituted or unsubstituted aralkyl having 7 to 50 carbon atoms, substituted or unsubstituted aryl group having 6 to 24 ring carbon atoms, and substituted or unsubstituted heteroaryl group having 5 to 30 ring atoms represented by R₁, R₂, Rₐ, R_{b} and R_{c} are the same as those described above.

The substituted or unsubstituted alkenyl group having 2 to 25 carbon atoms and substituted or unsubstituted alkynyl group having 2 to 25 carbon atoms represented by R₁, R₂, Rₐ, R_{b} and R_{c} are the same as those described above.

The mono-anionic bidentate ligand represented by L' is exemplified by a ligand represented by a formula (L') below.

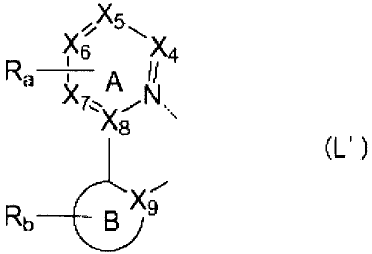

In the formula (L'), X₄ to X₉, Rₐ and R_{b} are the same as X₄ to X₉, Rₐ and R_{b} in the formula (T). Preferable examples of X₄ to X₉, Rₐ and R_{b} in the formula (L') are the same as those in the formula (T).

The ligand represented by the formula (L') is coordinated with the metal M represented in the formula (T) through a solid line extending from Xg to an outside of the ring B and a dashed line extending from a nitrogen atom of the ring A to an outside of the ring A.

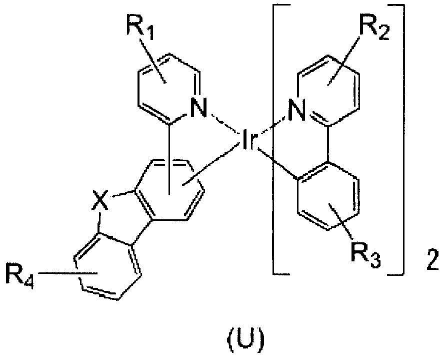

In the formula (U), X represents one selected from the group consisting of NR, an oxygen atom, a sulfur atom, BR, and a selenium atom. R represents a hydrogen atom or a substituted or unsubstituted alkyl group having 1 to 25 carbon atoms.

R₁, R₂, R₃ and R₄ each independently represent one selected from the group consisting of a hydrogen atom, a substituted or unsubstituted alkyl group having 1 to 25 carbon atoms and a substituted or unsubstituted aryl group having 6 to 24 ring carbon atoms. The number of R₁, R₂, R₃ and R₄ each independently ranges from 1 to 4.

In the formula (U), the alkyl group having 1 to 25 carbon atoms represented by R, R₁, R₂, R₃ and R₄ is exemplified by the above-described groups. Preferable examples of the alkyl group having 1 to 25 carbon atoms represented by R, R₁, R₂, R₃ and R₄ are the same as those described above.

Moreover, the aryl group having 6 to 24 ring carbon atoms represented by R₁, R₂, R₃ and R₄ is exemplified by the above-described groups. Preferable examples of the aryl group having 6 to 24 ring carbon atoms represented by R₁, R₂, R₃ and R₄ are the same as those described above.

Preferred complexes of the formula (T) or (U) are shown below, but are not limited thereto.

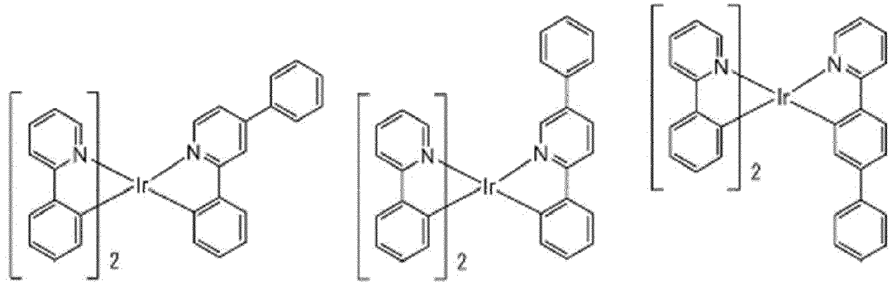

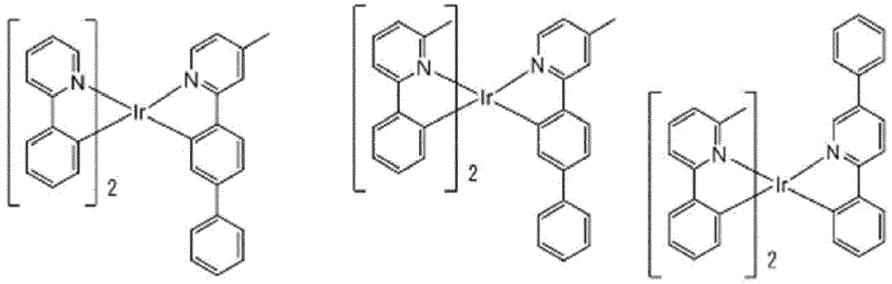

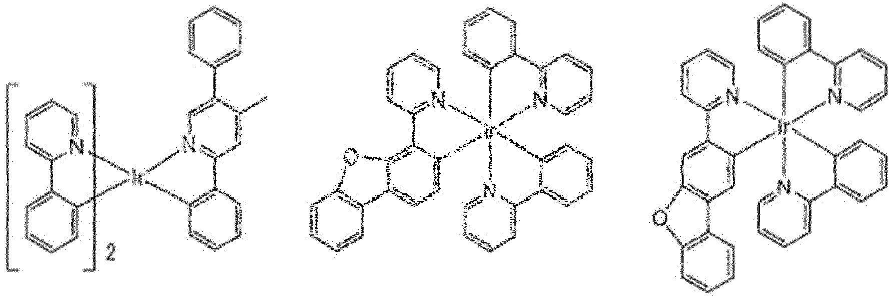

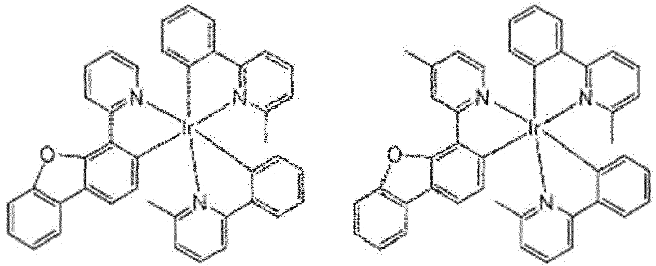

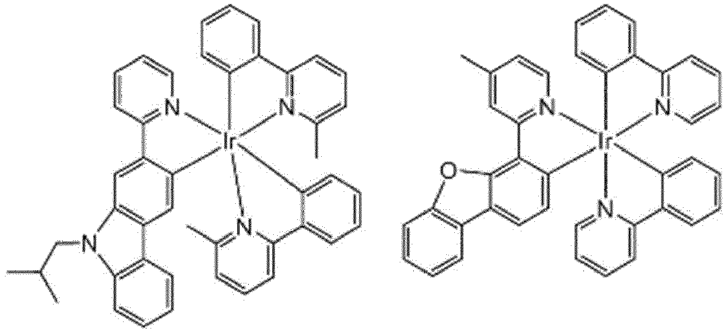

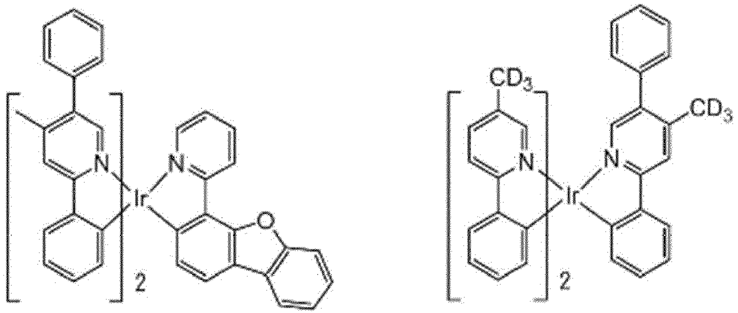

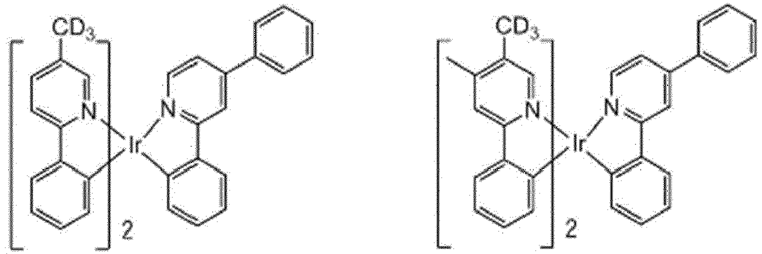

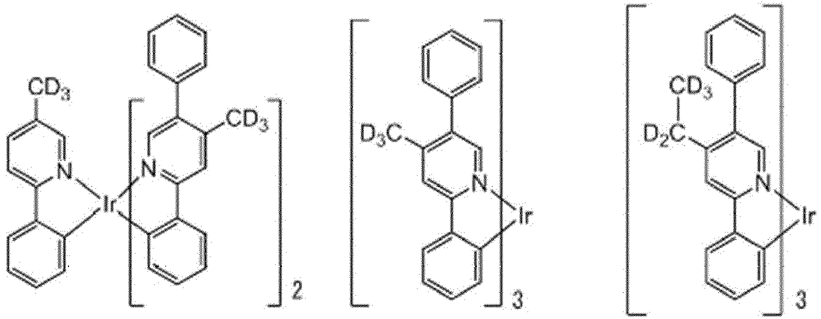

The complex represented by the formula (α) may be a complex represented by a formula (V), (X), (Y) or (Z) below in addition to the complex represented by the formula (T) or (U).

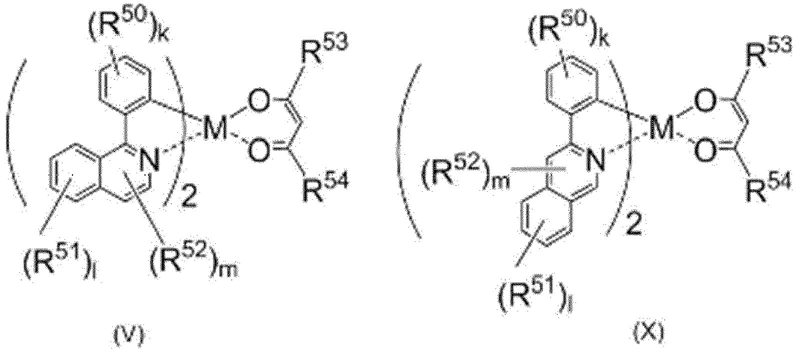

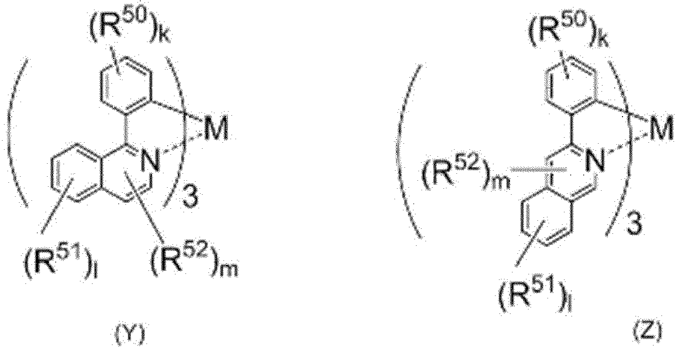

In the formula (V), (X), (Y) or (Z), R⁵⁰ to R⁵⁴ each independently represent a hydrogen atom or a substituent, k is an integer of 1 to 4, l is an integer of 1 to 4, and m is an integer of 1 to 2. M is Ir, Os, or Pt.

Examples of the substituent represented by R⁵⁰ to R⁵⁴ are the same substituent as Rₐ, R_{b} and R_{c} in formula (T) described above.

The formula (V) is preferably represented by a formula (V-1). The formula (X) is preferably represented by a formula (X-1) or (X-2). In the following formulae (V-1), (X-1) and (X-2), R⁵⁰, k and M represent the same as R⁵⁰, k and M described above.

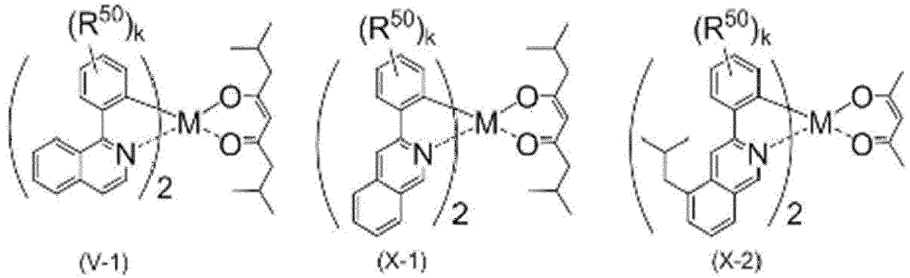

Examples of the complex represented by the formula (V), (X), (Y) or (Z) are shown below, but the complex is not limited to the examples.

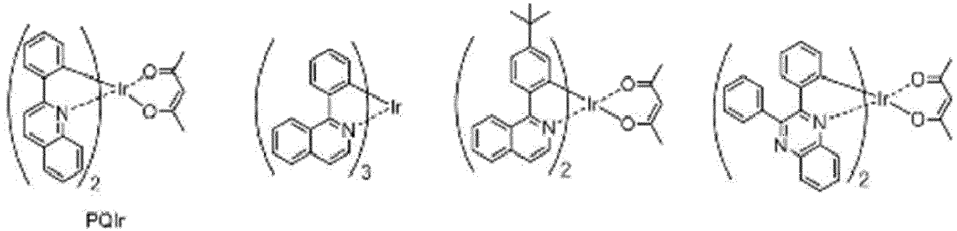

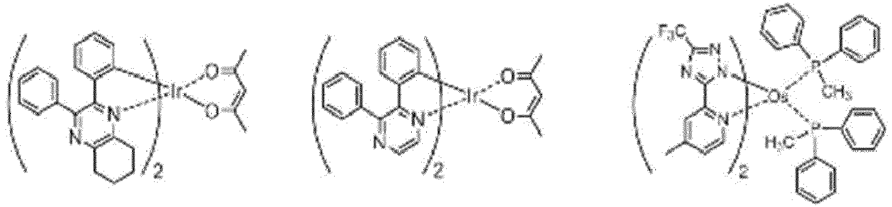

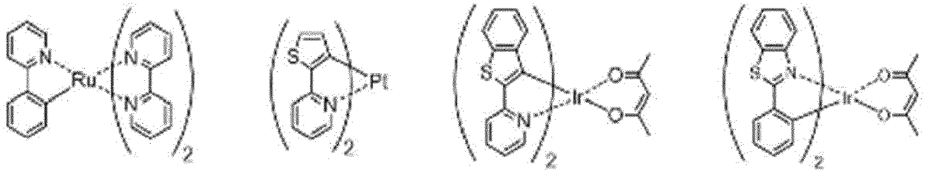

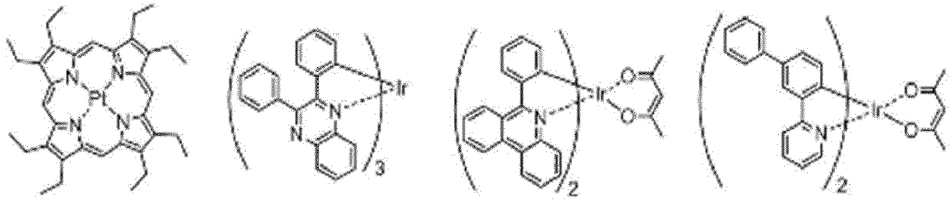

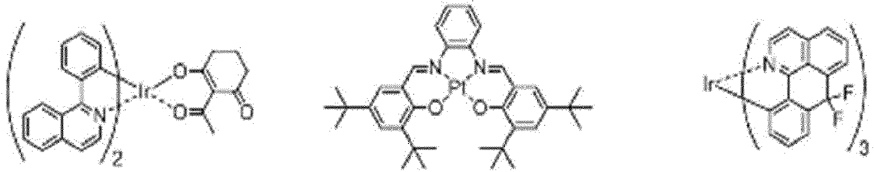

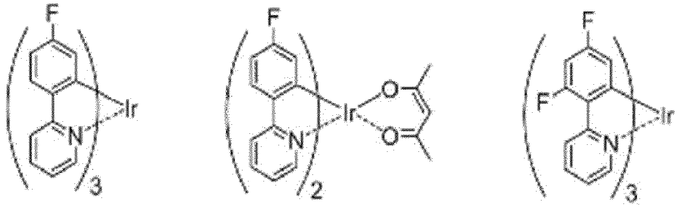

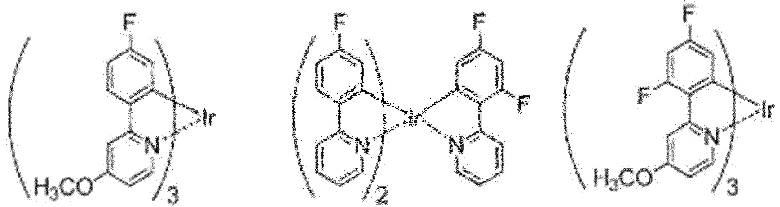

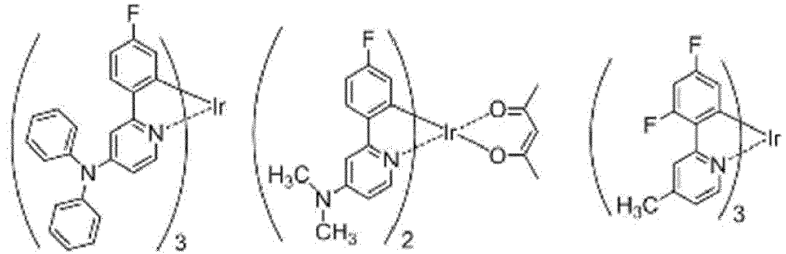

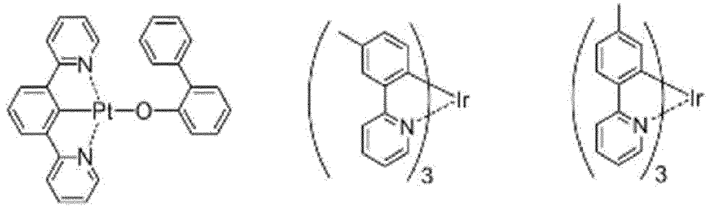

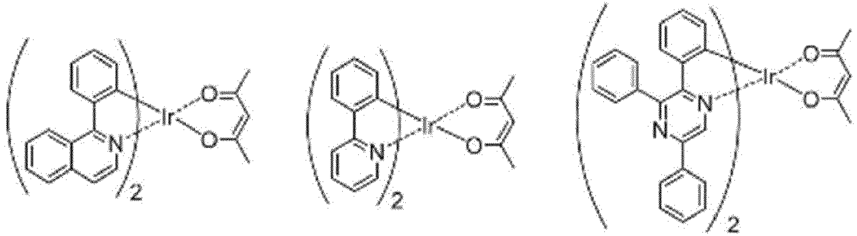

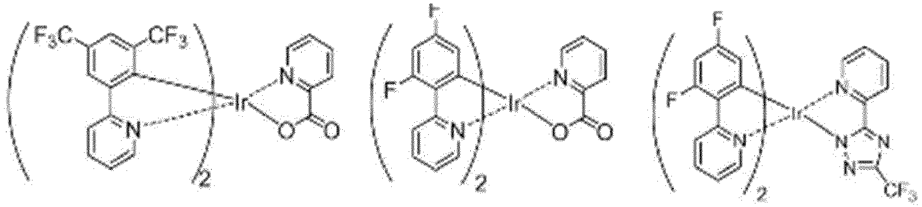

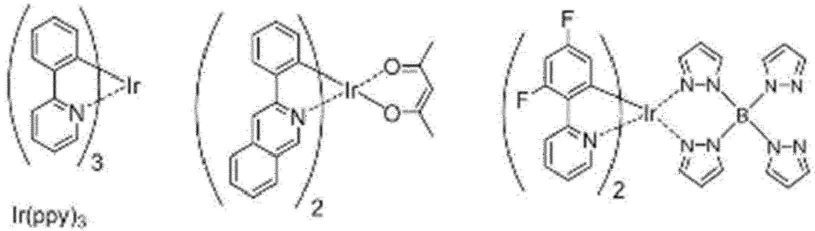

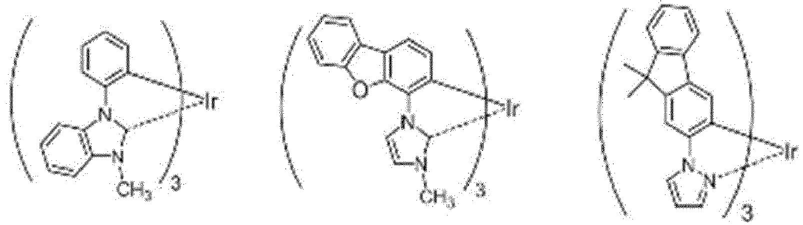

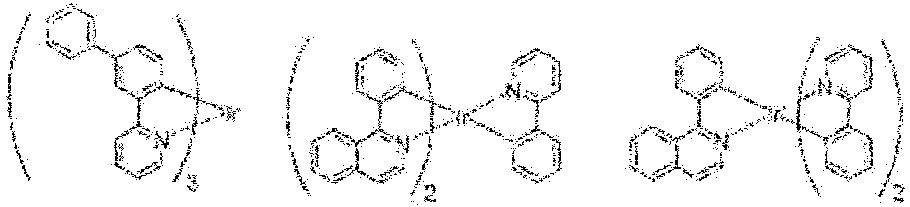

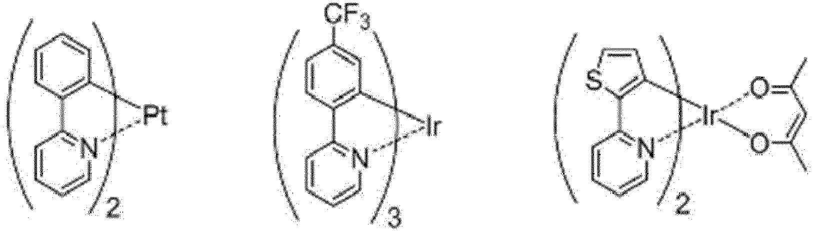

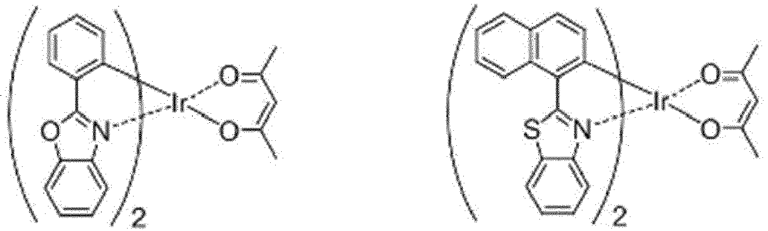

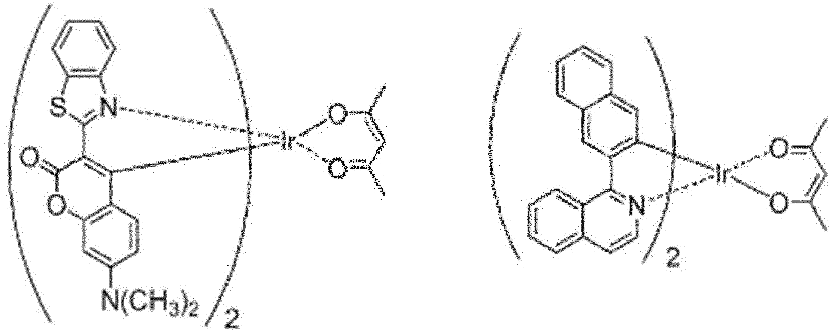

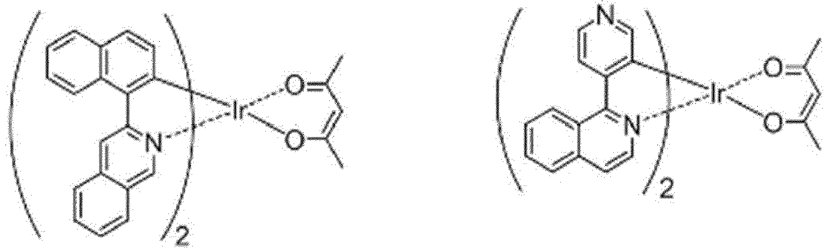

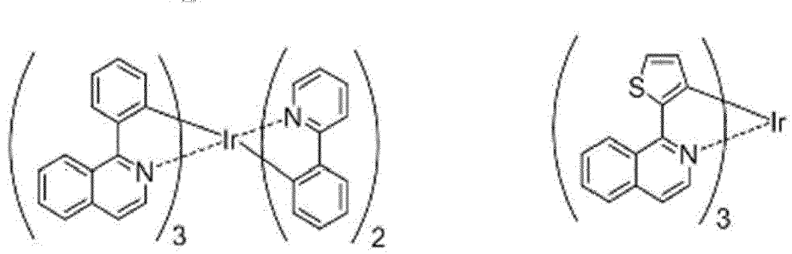

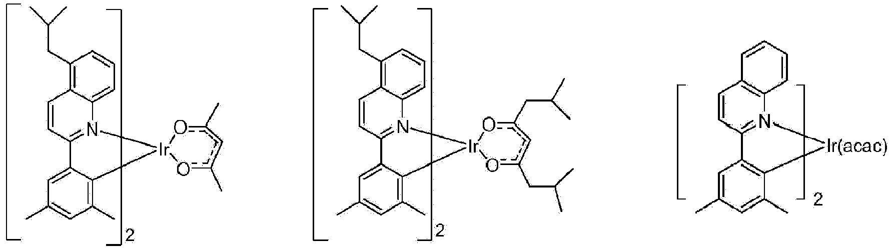

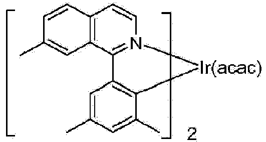

The phosphorescent material is also preferably an iridium complex represented by a formula (β) below.

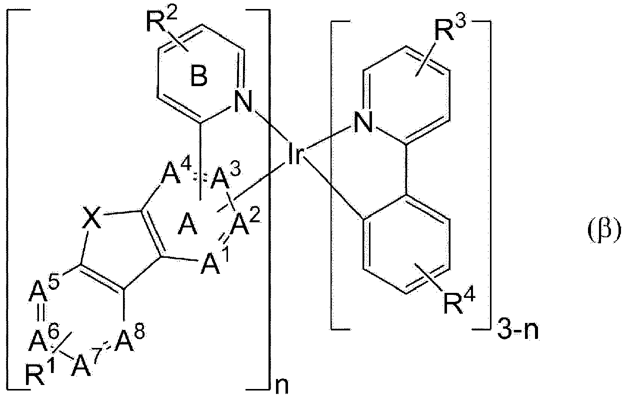

In the formula (β), A₁ to A⁸ include a carbon atom or a nitrogen atom, at least one of A¹ to A⁸ is a nitrogen atom, the ring B is bonded to the ring A by C-C bond, and iridium (Ir) is bonded to the ring A by Ir-C bond.

In the formula (β), only one of A¹ to A⁸ is preferably a nitrogen atom, more preferably only one of A⁵ to A⁸ is a nitrogen atom, and further preferably A⁵ is a nitrogen atom. In the formula (β), A³ and A⁴ of A¹ to A⁴ are preferably carbon atoms. In the formula (β), it is preferable that A⁵ is a nitrogen atom and A¹ to A⁴ and A⁶ to A⁸ are carbon atoms.

A⁶ is preferably CR (carbon atom bonded with R), in which R is selected from the group consisting of a substituted or unsubstituted alkyl group having 1 to 25 carbon atoms, a substituted or unsubstituted cycloalkyl group having 3 to 25 ring carbon atoms, and a combination thereof. R is preferably a substituted or unsubstituted alkyl group having 1 to 25 carbon atoms or a substituted or unsubstituted cycloalkyl group having 3 to 25 ring carbon atoms.

In the formula (β), X is O, S or Se, preferably O.

In the formula (β), R¹ to R⁴ are each independently mono-substituted, di-substituted, trisubstituted, tetra-substituted, or unsubstituted. Adjacent ones of R¹ to R⁴ are optionally bonded to each other to form a ring. R¹ to R⁴ are each independently selected from the group consisting of hydrogen, deuterium, halogen, a substituted or unsubstituted alkyl group having 1 to 25 carbon atoms, a substituted or unsubstituted cycloalkyl group having 3 to 25 ring carbon atoms, a substituted or unsubstituted heteroalkyl group having 2 to 25 atoms, a substituted or unsubstituted arylalkyl group having 7 to 50 carbon atoms, a substituted or unsubstituted alkoxy group having 1 to 25 carbon atoms, a substituted or unsubstituted aryloxy group having 6 to 24 ring carbon atoms, a substituted or unsubstituted amino group, a substituted silyl group, a substituted or unsubstituted alkenyl group having 2 to 25 carbon atoms, a cycloalkenyl group having 3 to 25 ring carbon atoms, a heteroalkenyl group having 3 to 25 atoms, an alkynyl group having 2 to 25 carbon atoms, an aryl group having 6 to 24 ring carbon atoms, a heteroaryl group having 5 to 30 ring atoms, an acyl group, a substituted carbonyl group, carboxyl group, ester, a nitrile group, an isonitrile group, a sulfanyl group, a sulfinyl group, a sulfonyl group, a phosphoryl group, and a combination thereof. The substituted silyl group is a silyl group substituted by at least one group selected from the group consisting of an alkyl group having 1 to 25 carbon atoms and an aryl group having 6 to 24 ring carbon atoms. The substituted carbonyl group is a carbonyl group substituted by at least one group selected from the group consisting of an alkyl group having 1 to 25 carbon atoms and an aryl group having 6 to 24 ring carbon atoms. In the formula (β), it is preferable that R¹ to R⁴ are each independently selected from the group consisting of hydrogen, deuterium, an alkyl group having 1 to 25 carbon atoms, and a combination thereof. At least one of R² and R³ is preferably an alkyl group having 1 to 25 carbon atoms, in which the alkyl group may be deuterated or partially deuterated.

In the formula (β), n is an integer of 1 to 3, preferably 1.

Examples of the iridium complex represented by the formula (β) are shown below, but the iridium complex is not limited to the examples.

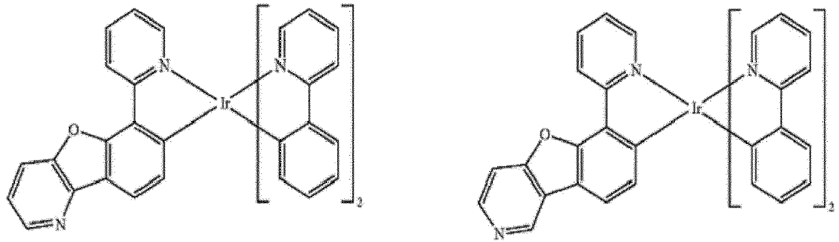

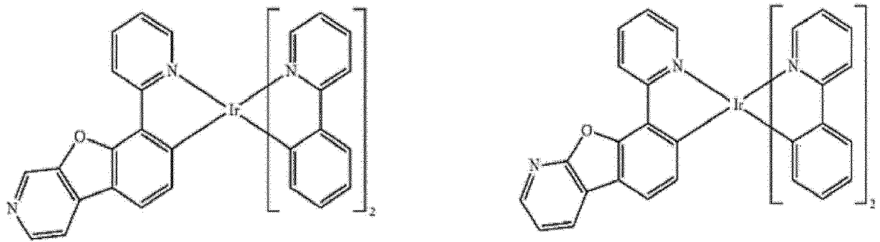

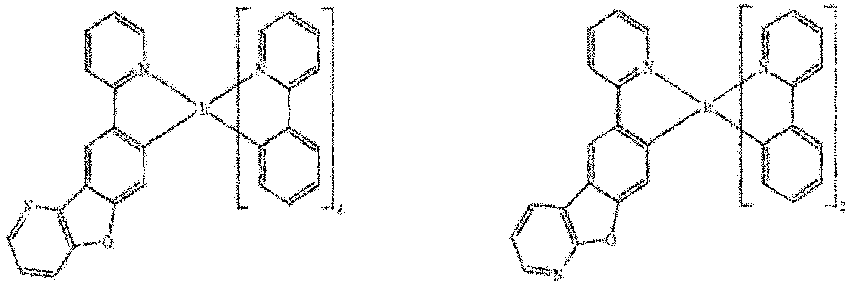

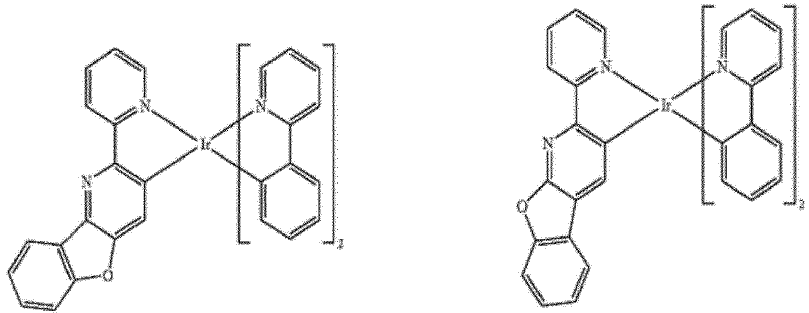

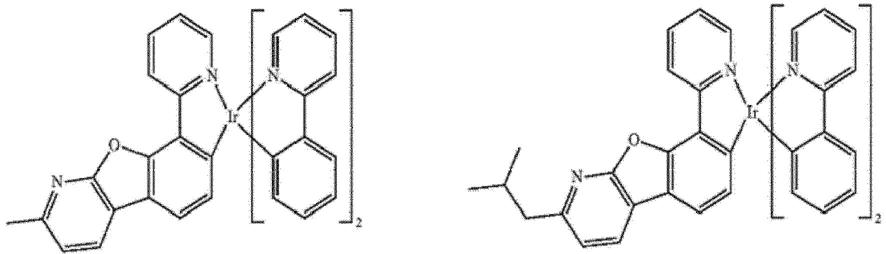

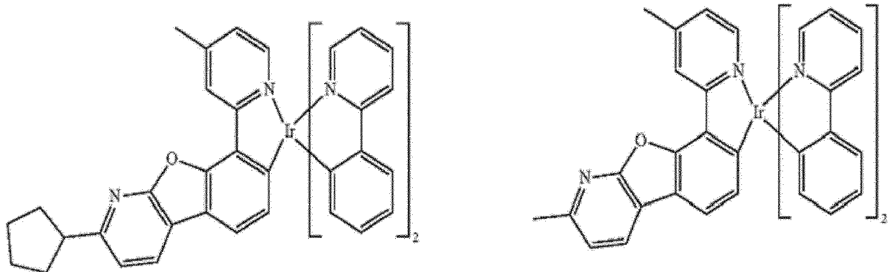

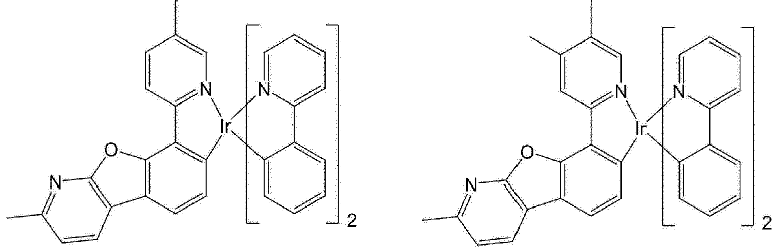

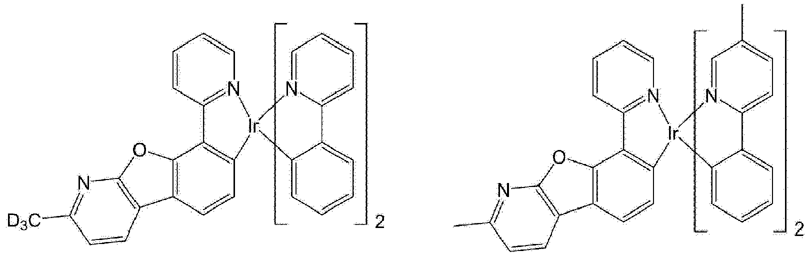

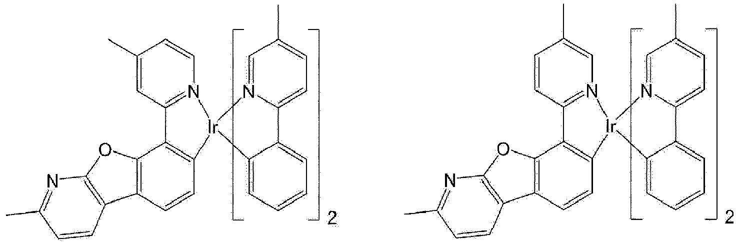

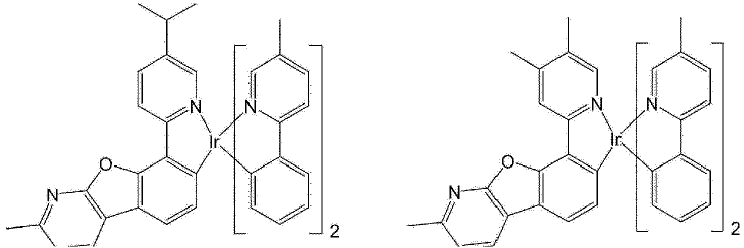

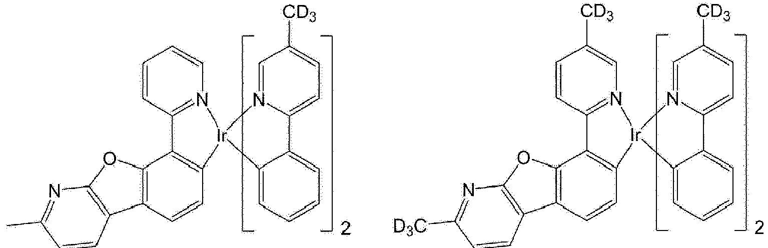

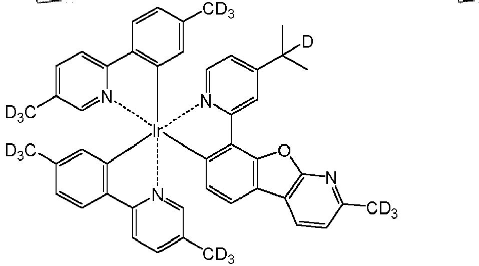

### Host Material in the Phosphorescent Emitting Layer

The host material used in the phosphorescent layer is preferably a material having a higher triplet level than a phosphorescent dopant. A general phosphorescent host material such as an aromatic compound, a heterocyclic compound, and a metal complex is usable as the host material used in the phosphorescent layer. Among the examples of the host material used in the phosphorescent layer, an aromatic compound and a heterocyclic compound are preferable. Examples of the aromatic compound include a naphthalene compound, triphenylene compound, phenanthrene compound, and a fluoranthene compound. Examples of the heterocyclic compound include an indole compound, carbazole compound, pyridine compound, pyrimidine compound, triazine compound, quinoline compound, isoquinoline compound, quinazoline compound, dibenzofuran compound, and dibenzothienyl compound. Herein, the compound is defined the same as described above.

Preferably, the composition of the present invention is employed in the emitting layer, more preferably as host material. The emitting layer of the organic EL device according to the present invention therefore more preferably comprises the composition according to the present invention and further comprises a luminescent material. Suitable luminescent materials are mentioned above. Most preferably, the luminescent material is a phosphorescent material, preferably an ortho-metalated complex of a metal atom selected from the group consisting of iridium, osmium and platinum. Suitable phosphorescent materials are mentioned above. The composition of the present invention is therefore most preferably employed in the phosphorescent emitting layer of an organic EL device. The organic EL device according to the present invention therefore preferably comprises an emitting layer comprising a phosphorescent dopant and the composition according to the present invention as a host. More preferably, the phosphorescent dopant emits a green phosphorescence, preferably having a main peak wavelength (occasionally referred to as an emission peak wavelength) of 490 to 570 nm.

### Electron Transporting Layer

The electron transporting layer is a layer containing a highly electron-transporting substance.

At least one layer may be provided between the electron transporting layer and the emitting layer in order to improve the performance of the organic EL device. The at least one layer is referred to as a second electron transporting layer, hole blocking layer or triplet block layer. In order to improve hole blocking capability, a material having a deep HOMO level is preferably used. In order to improve triplet block capability, a material having a high triplet level is preferably used.

In the electron transporting layer, a metal complex such as an aluminum complex, beryllium complex and zinc complex; a hetero cyclic compound such as an imidazole compound, benzimidazole compound, azine compound, carbazole compound, and phenanthroline compound; a fused aromatic hydrocarbon compound; and a high-molecular compound are usable. Preferred examples of the material for the electron transporting layer include an imidazole compound (e.g., a benzimidazole compound, imidazopyridine compound, and benzimidazophenanthridine compound), an azine compound (e.g., a pyrimidine compound, triazine compound, quinoline compound, isoquinoline compound, and phenanthroline compound (i.e., a hetero ring), which may be substituted by a phosphine oxide substituent), and an aromatic hydrocarbon compound (e.g., an anthracene compound and fluoranthene compound).

In one preferred embodiment of the present invention, the electron transporting layer, the hole blocking layer or the triplet block layer comprise the composition of the present invention.

In a further preferred embodiment, the electron transporting layer may include at least one selected from the group consisting of an alkali metal (e.g., Li and Cs), an alkaline earth metal (e.g., Mg), an alloy thereof, a compound of the alkali metal (e.g., a lithium quinolinato complex), and a compound of the alkaline earth metal. When the electron transporting layer includes at least one of the alkali metal, the alkaline earth metal, and the alloy thereof, a content ratio of the at least one included in the electron transporting layer is preferably in a range from 0.1 to 50 mass%, more preferably in a range from 0.1 to 20 mass%, further preferably in a range from 1 to 10 mass%. When the electron transporting layer includes at least one of the compound of the alkali metal and the compound of the alkaline earth metal, a content ratio of the at least one compound included in the electron transporting layer is preferably in a range from 1 to 99 mass%, more preferably in a range from 10 to 90 mass%.

### Electron Injecting Layer

The electron injecting layer is a layer containing a highly electron-injectable substance. Examples of a material for the electron injecting layer include an alkali metal, an alkaline earth metal, and an alloy thereof, examples of which include lithium (Li), lithium fluoride (LiF), cesium fluoride (CsF), calcium fluoride (CaF₂), and lithium oxide (LiOx), a compound of the alkali metal (e.g., a lithium quinolinato complex), and a compound of the alkaline earth metal.

### Cathode

A metal, an alloy, an electrically conductive compound, a mixture thereof and the like, which have a small work function, specifically, of 3.8 eV or less, is preferably usable as a material for the cathode. Examples of the material for the cathode include elements belonging to Groups 1 and 2 in the periodic table of the elements, specifically, an alkali metal such as lithium (Li) and cesium (Cs), an alkaline earth metal such as magnesium (Mg), and an alloy (e.g., MgAg and AILi) including the alkali metal or the alkaline earth metal, a rare earth metal, and an alloy including the rare earth metal.

### Electronic Device

The present invention further relates to an electronic device comprising the organic electroluminescence device according to the present invention.

The organic electroluminescence device of the present invention is usable for various electronic devices. For example, the organic electroluminescence device of the present invention is usable for a light source of a flat light-emitting body, a backlight, instruments and the like, a display plate, sign lamp and the like. The flat light-emitting body is exemplified by a flat panel display of a wall-hanging TV. The backlight is exemplified by a backlight of a copier, a printer, a liquid crystal display and the like.

Moreover, the composition according to the present invention is usable not only in an organic EL device but also in fields such as an electrophotographic photoreceptor, photoelectric conversion element, solar battery and image sensor.

### Modification of Embodiments

It should be noted that the invention is not limited to the above exemplary embodiments but may include any modification and improvement as long as such modification and improvement are compatible with the invention.

In the description above, the inventive composition preferably being contained in the emitting layer is described. An organic EL device according to still another embodiment comprises a composition in a single layer of the organic layer(s) except for the emitting layer. For instance, an organic EL device comprises an anode, a cathode, an emitting layer provided between the anode and the cathode, and an electron transporting zone provided between the emitting layer and the cathode, wherein the electron transporting zone comprises the composition of the present invention.

Further, the emitting layer is not limited to a single layer, but may be provided by laminating a plurality of emitting layers. When the organic EL device has a plurality of emitting layers, it is only required that at least one of the emitting layers satisfies the conditions described in the above. For instance, the rest of the emitting layer may be a fluorescent emitting layer or a phosphorescent emitting layer which do not comprise the composition according to the present invention, for example a phosphorescent emitting layer with use of emission by electron transfer from the triplet state directly to the ground state.

When the organic EL device includes a plurality of emitting layers, the plurality of emitting layers may be adjacent to each other, or provide a so-called tandem-type organic EL device in which a plurality of emitting units are layered through an intermediate layer.

For instance, a blocking layer may be provided adjacent to at least one side of a side near the anode and a side near the cathode of the emitting layer. The blocking layer is preferably provided in contact with the emitting layer to at least block holes, electrons or excitons.

For instance, when the blocking layer is provided in contact with the cathode-side of the emitting layer, the blocking layer permits transport of electrons, but blocks holes from reaching a layer provided near the cathode (e.g., the electron transporting layer) beyond the blocking layer. When the organic EL device includes an electron transporting layer, the blocking layer is preferably interposed between the emitting layer and the electron transporting layer.

When the blocking layer is provided in contact with the anode-side of the emitting layer, the blocking layer permits transport of holes, but blocks electrons from reaching a layer provided near the anode (e.g., the hole transporting layer) beyond the blocking layer. When the organic EL device includes a hole transporting layer, the blocking layer is preferably interposed between the emitting layer and the hole transporting layer.

Further, the blocking layer may be provided in contact with the emitting layer to prevent an excitation energy from leaking from the emitting layer into neighboring layers. The blocking layer blocks excitons generated in the emitting layer from moving into a layer provided near the electrode (e.g., the electron transporting layer and the hole transporting layer) beyond the blocking layer.

The emitting layer is preferably in contact with the blocking layer.

Specific structure and shape of the components in the present invention may be designed in any manner as long as the object of the present invention can be achieved.

### Example(s)

Example(s) of the invention will be described below. However, the invention is not limited to the example(s).

### I Synthesis Examples

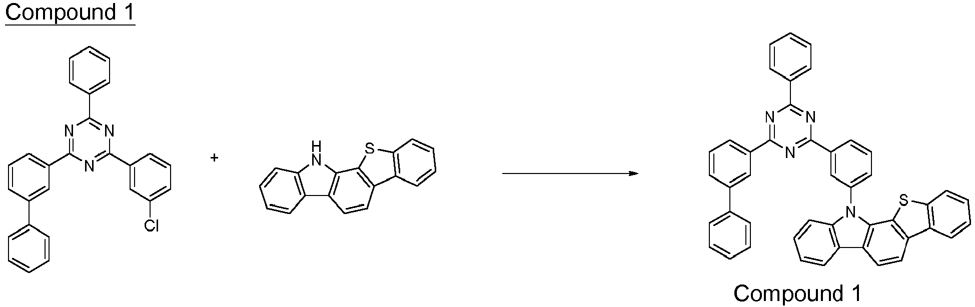

6.84g (25.0mmol) 12H-benzo[4,5]thieno[2,3-a]carbazole (commercially available), 7.0g (16.7mmol) 2-([1,1'-biphenyl]-3-yl)-4-(3-chlorophenyl)-6-phenyl-1,3,5-triazine (synthesized according to EP3287505, intermediate 1-12) and 6.61g (66.7mmol) sodium t-butoxide were dissolved in 200ml toluene. The reaction mixture was evacuated and backfilled with argon three times. 0.8g (2.67mmol) tri-tert-butylphosphonium tetrafluoroborate and 0.63g (0.67mmol) Pd₂(dba)₃ were added and the reaction mixture was evacuated and backfilled with argon three times. The reaction mixture was heated to reflux for 20h, then cooled to RT. 150ml toluene were distilled off on the rotavap and the resulting suspension was filtered. The residue was washed with toluene, water and methanol and dried at 80°C/125mbar overnight to yield 10.4g of crude product, that was crystallized from DMF to yield 5.77g (53%) of Compound 1: 12-(3-(4-([1,1'-biphenyl]-3-yl)-6-phenyl-1,3,5-triazin-2-yl)phenyl)-12H-benzo[4,5]thieno[2,3-a]carbazole as a slightly yellow solid.
¹H NMR (400 MHz, Chloroform-*d*) δ 9.09 (dt, *J* = 7.8, 1.5 Hz, 1H), 9.06 (t, *J* = 1.9 Hz, 1H), 8.97 (t, *J* = 1.8 Hz, 1H), 8.79 - 8.69 (m, 3H), 8.32 - 8.21 (m, 3H), 8.14 (d, *J* = 8.3 Hz, 1H), 7.89 (t, *J* = 7.7 Hz, 1H), 7.86 - 7.77 (m, 2H), 7.72 - 7.65 (m, 3H), 7.63 - 7.31 (m, 12H).

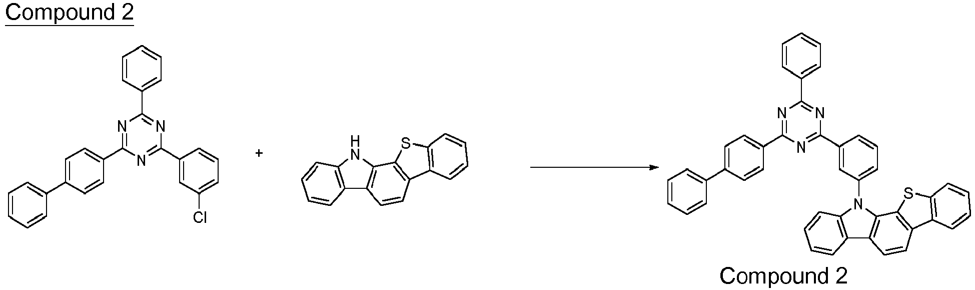

Prepared the same way as described for compound 1 but using 2-([1,1'-biphenyl]-4-yl)-4-(3-chlorophenyl)-6-phenyl-1,3,5-triazine (commercially available) instead of 2-([1,1'-biphenyl]-3-yl)-4-(3-chlorophenyl)-6-phenyl-1,3,5-triazine to yield Compound 2: 12-(3-(4-([1,1'-biphenyl]-4-yl)-6-phenyl-1,3,5-triazin-2-yl)phenyl)-12H-benzo[4,5]thieno[2,3-a]carbazole.
¹H NMR (300 MHz, Tetrachloroethane-*d*₂) δ 9.18 (dt, *J* = 7.8, 1.4 Hz, 1H), 9.11 (t, *J* = 1.8 Hz, 1H), 8.94 - 8.76 (m, 4H), 8.42 - 8.27 (m, 3H), 8.22 (d, *J* = 8.3 Hz, 1H), 8.00 (t, *J* = 7.7 Hz, 1H), 7.92 (dt, *J* = 7.9, 1.4 Hz, 1H), 7.88 - 7.81 (m, 2H), 7.81 - 7.70 (m, 3H), 7.70 - 7.38 (m, 11H).

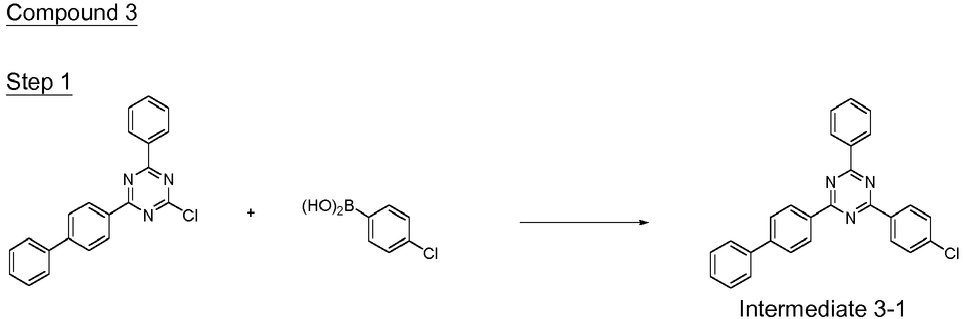

Intermediate 3-1: 2-([1,1'-biphenyl]-4-yl)-4-(4-chlorophenyl)-6-phenyl-1,3,5-triazine was prepared using the conditions described in EP3287505 for intermediate 1-12 but using 2-([1,1'-biphenyl]-4-yl)-4-chloro-6-phenyl-1,3,5-triazine (commercially available) and (4-chlorophenyl)boronic acid (commercially available) as starting materials.
¹H NMR (400 MHz, Chloroform-d) δ 8.86 - 8.66 (m, 6H), 7.84 - 7.75 (m, 2H), 7.75 - 7.68 (m, 2H), 7.68 - 7.47 (m, 7H), 7.47 - 7.39 (m, 1H).

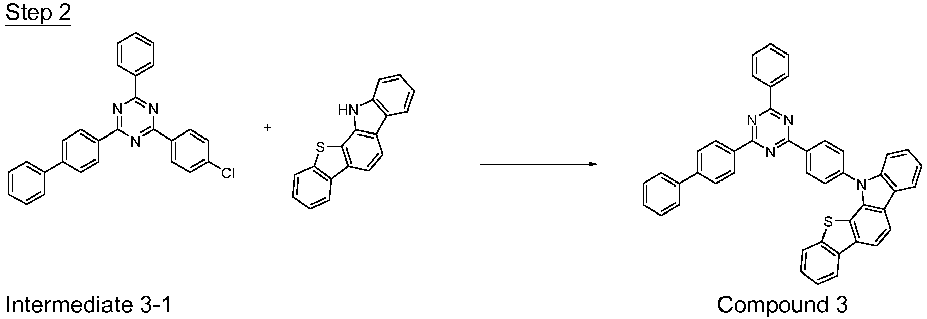

Prepared the same way as described for compound 1 but using Intermediate 3-1: 2-([1,1'-biphenyl]-4-yl)-4-(4-chlorophenyl)-6-phenyl-1,3,5-triazine instead of 2-([1,1'-biphenyl]-3-yl)-4-(3-chlorophenyl)-6-phenyl-1,3,5-triazine to yield Compound 3: 12-(4-(4-([1,1'-biphenyl]-4-yl)-6-phenyl-1,3,5-triazin-2-yl)phenyl)-12H-benzo[4,5]thieno[2,3-a]carbazole.
¹H NMR (400 MHz, Chloroform-*d*) δ 9.17 - 9.07 (m, 2H), 8.95-8.81 (m, 4H), 8.31 - 8.19 (m, 3H), 8.12 (d, *J* = 8.3 Hz, 1H), 7.88 - 7.79 (m, 4H), 7.73 (ddd, *J* = 8.2, 3.2, 1.2 Hz, 3H), 7.70 - 7.58 (m, 3H), 7.56 - 7.33 (m, 8H).

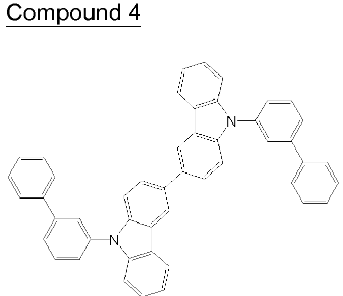

Synthesized according to WO2015190718, example C-2

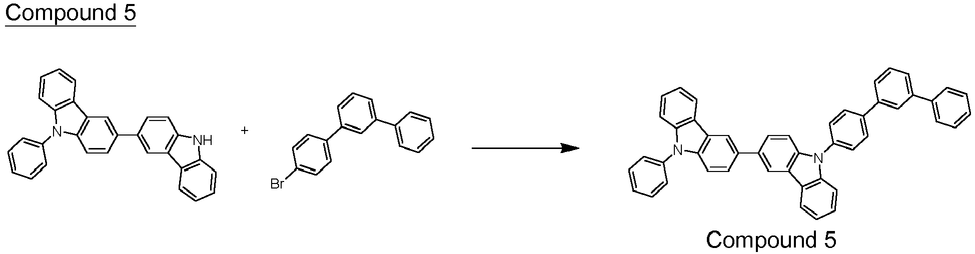

Synthesized the same as synthesis example 2-1-2 in US20130313536, but using 4-bromo-1,1':3',1"-terphenyl (commercially available) instead of 4'-bromobiphenyl-4-carbonitrile to yield Compound 5: 9-([1,1':3',1"-terphenyl]-4-yl)-9'-phenyl-9H,9'H-3,3'-bicarbazole.
¹H NMR (300 MHz, Tetrachloroethane-*d*₂) δ 8.53 (t, *J* = 1.6 Hz, 2H), 8.37 - 8.28 (m, 2H), 8.06 - 7.96 (m, 3H), 7.89 (ddd, *J* = 8.6, 5.2, 1.8 Hz, 2H), 7.79 (dt, *J* = 8.1, 1.8 Hz, 5H), 7.70 (td, *J* = 7.4, 6.8, 3.8 Hz, 7H), 7.66 - 7.35 (m, 11H).

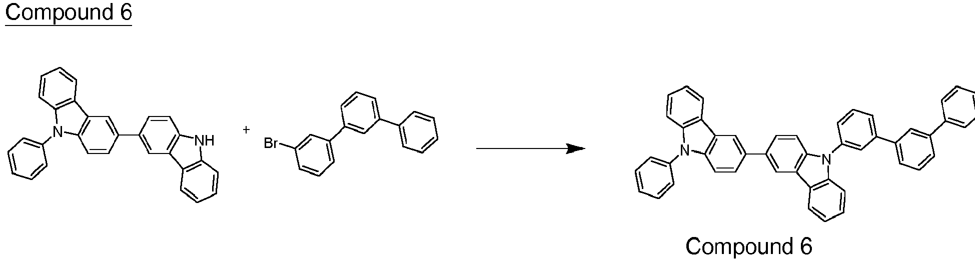

Synthesized the same as synthesis example 2-1-2 in US20130313536, but using 3-bromo-1,1':3',1"-terphenyl (commercially available) instead of 4'-bromobiphenyl-4-carbonitrile to yield Compound 6: 9-([1,1':3',1"-terphenyl]-3-yl)-9'-phenyl-9H,9'H-3,3'-bicarbazole.
¹H NMR (300 MHz, Tetrachloroethane-*d*₂) δ 8.55 (dd, *J* = 3.7, 1.7 Hz, 2H), 8.38 - 8.30 (m, 2H), 8.00 (dt, *J* = 8.8, 1.9 Hz, 2H), 7.89 (ddd, *J* = 8.3, 3.9, 1.7 Hz, 3H), 7.82 (t, *J* = 7.7 Hz, 1H), 7.78 - 7.67 (m, 10H), 7.66 - 7.37 (m, 12H).

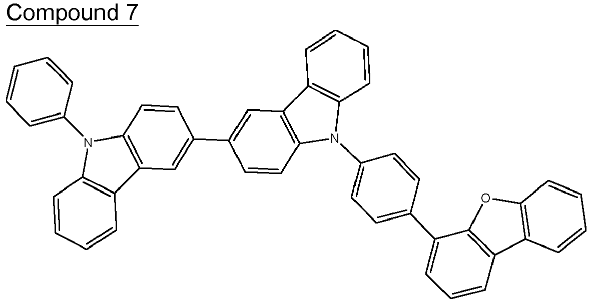

Synthesized according to EP 2674429, example 1-1

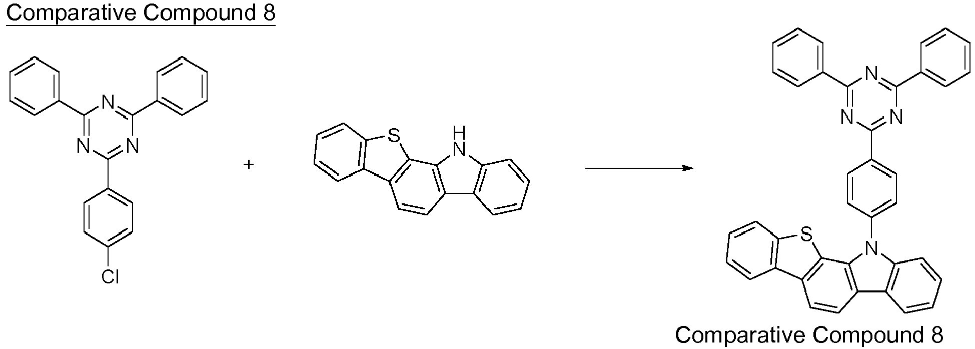

Prepared the same way as described for Compound 1 but using 2-(4-chlorophenyl)-4,6-diphenyl-1,3,5-triazine (commercially available) instead of 2-([1,1'-biphenyl]-3-yl)-4-(3-chlorophenyl)-6-phenyl-1,3,5-triazine to yield Comparative Compound 8: 12-(4-(4,6-diphenyl-1,3,5-triazin-2-yl)phenyl)-12H-benzo[4,5]thieno[2,3-a]carbazole.
¹H NMR (300 MHz, Tetrachloroethane-*d*₂) δ 9.23 - 9.12 (m, 2H), 8.95 - 8.83 (m, 4H), 8.39 - 8.27 (m, 3H), 8.21 (d, *J* = 8.3 Hz, 1H), 7.96 - 7.87 (m, 2H), 7.86 - 7.79 (m, 1H), 7.79 - 7.63 (m, 6H), 7.61 - 7.37 (m, 5H).

### II Application Examples

### Application example 1

0.2g of Compound 1 was measured. 0.2g of Compound 4 was measured. The two compounds were mixed in a mortar. After being mixed, 0.2g of the obtained mixture was fed into a quartz crucible for a vacuum evaporator. A part of the mixture of the two compounds in the crucible was collected. The collected substance was dissolved in tetrahydrofuran. Based on a peak area value of each of components detected by HPLC (High Performance Liquid Chromatography), a ratio (initial ratio) between the two compounds was measured to be of: 48.2 : 51.8.

The crucible filled with the two compounds was set in the vacuum evaporator and the mixture was evaporated onto a glass substrate until 80% of the original content in the crucible was deposited on the glass substrate.

Lastly, the residue in the crucible was taken out of the evaporator and was dissolved in tetrahydrofuran. Based on a peak area value of each of components detected by HPLC, the ratio (residual ratio) between the two compounds was calculated.
Table 1 showed the ratio (initial ratio) between the first compound and the second compound in the crucible in the initial stage (before the evaporation) and the ratio (residual ratio) between the first compound and the second compound in the crucible after evaporation.

### Application example 2

Application example 2 was manufactured in the same manner as the Application example 1 except for using Compound 2 and Compound 5.

### Application example 3

Application example 3 was manufactured in the same manner as the Application example 1 except for using Compound 1 and Compound 6.

### Application example 4

Application example 4 was manufactured in the same manner as the Application example 1 except for using Compound 3 and Compound 7.

### Comparative application example 1

Comparative application example 1 was manufactured in the same manner as the Application example 1 except for using Comparative Compound 8 and Compound 4.

**Table 1**

| | Compound (1) | Compound (2) | Material Ratio in Crucible | |
|---|---|---|---|---|
| | | | Initial Ratio | Residual Ratio |
| Appl. Exp. 1 | Compound 1 | Compound 4 | 48.2 : 51.8 | 46.4 : 53.6 |
| Appl. Exp. 2 | Compound 2 | Compound 5 | 51.7 : 48.3 | 50.6 : 49.4 |
| Appl. Exp. 3 | Compound 1 | Compound 6 | 49.6 : 50.4 | 48.4 : 51.6 |
| Appl. Exp. 4 | Compound 3 | Compound 7 | 48.7 : 51.3 | 49.6 : 50.4 |
| Comp. Appl. Exp. 1 | Compound 8 | Compound 4 | 64.6 : 35.4 | 35.5 : 64.5 |

These results clearly showed that in the application examples 1 to 4, the ratio of the evaporated compounds stayed constant whereas in the comparative example, the ratio of the evaporated compounds changed over time. This led to an undesired shift in device performance over time.

## Claims

1. A composition comprising
(i) a compound of formula (1) 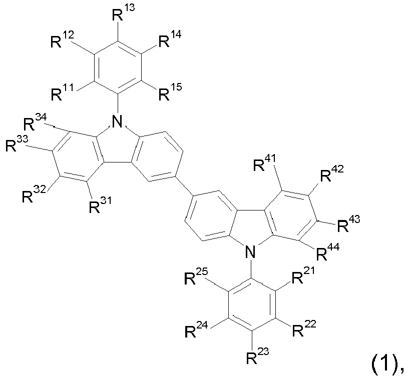 and
(ii) a compound of formula (2) 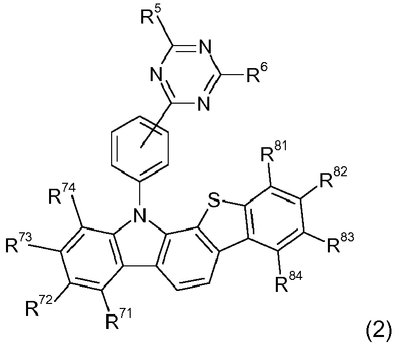 wherein
R¹¹ R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ each independently represent hydrogen, CN, an unsubstituted phenyl group, a phenyl group substituted by at least one CN group, an unsubstituted biphenyl group, a biphenyl group substituted by at least one CN group, an unsubstituted dibenzofuran group, a dibenzofuran group substituted by at least one CN group, an unsubstituted dibenzothiophene group or a dibenzothiophene group substituted by at least one CN group, wherein the sum of ring carbon atoms of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ is exactly 12;
R⁵ represents an unsubstituted phenyl group;
R⁶ represents an unsubstituted phenyl group, an unsubstituted biphenyl group, an unsubstituted dibenzofuran group or an unsubstituted dibenzothiophene group;
R⁷¹, R⁷², R⁷³, R⁷⁴, R⁸¹, R⁸², R⁸³ and R⁸⁴ each independently represent hydrogen or an unsubstituted phenyl group;
wherein the sum of ring carbon atoms of the residues R⁵, R⁶, R⁷¹, R⁷², R⁷³, R⁷⁴, R⁸¹, R⁸², R⁸³ and R⁸⁴ is exactly 18.

2. The composition according to claim 1, wherein
R¹¹, R¹², R¹³, R¹⁴, R¹⁵, R²¹, R²², R²³, R²⁴ and R²⁵ each independently represent hydrogen, CN, an unsubstituted phenyl group, a phenyl group substituted by at least one CN group, an unsubstituted biphenyl group, an unsubstituted dibenzofuran group, or an unsubstituted dibenzothiophene group; and
R³¹, R³², R³³, R³⁴, R⁴¹, R⁴², R⁴³ and R⁴⁴ each independently represent hydrogen, CN, an unsubstituted phenyl group, or a phenyl group substituted by at least one CN group.

3. The composition according to claim 1 or 2, wherein
one, two, three or four of the residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ have the following meanings:
R¹¹, R¹², R¹³, R¹⁴, R¹⁵, R²¹, R²², R²³, R²⁴ and R²⁵ each independently represent CN, an unsubstituted phenyl group, a phenyl group substituted by at least one CN, an unsubstituted biphenyl group, an unsubstituted dibenzofuran group, or an unsubstituted dibenzothiophene group, and
R³¹, R³², R³³, R³⁴, R⁴¹, R⁴², R⁴³ and R⁴⁴ each independently represent CN, an unsubstituted phenyl group, or a phenyl group substituted by at least one CN; and
the remaining residues R¹¹, R¹², R¹³, R¹⁴, R¹⁵, R²¹, R²², R²³, R²⁴, R²⁵, R³¹, R³², R³³, R³⁴, R⁴¹, R⁴², R⁴³ and R⁴⁴ are hydrogen.

4. The composition according to any one of claims 1 to 3, wherein one or two of residues R¹¹, R¹², R¹³, R¹⁴ and R¹⁵ and none, one or two of the residues R²¹, R²², R²³, R²⁴ and R²⁵ each independently represent CN, an unsubstituted phenyl group, a phenyl group substituted by at least one CN group, an unsubstituted biphenyl group, an unsubstituted dibenzofuran group, or an unsubstituted dibenzothiophene group, and the remaining residues R¹¹, R¹², R¹³, R¹⁴, R¹⁵, R²¹, R²², R²³, R²⁴ and R²⁵ are hydrogen.

5. The composition according to any one of claims 1 to 4, wherein R³¹, R³², R³³, R³⁴, R⁴¹, R⁴², R⁴³ and R⁴⁴ are hydrogen.

6. The composition according to any one of claims 1 to 5, wherein R⁷¹, R⁷², R⁷³, R⁷⁴, R⁸¹, R⁸², R⁸³ and R⁸⁴ are hydrogen.

7. An organic electroluminescence device material comprising the composition according to any one of claims 1 to 6.

8. A composition film comprising the composition according to any one of claims 1 to 6.

9. An organic electroluminescence device, comprising:
an anode;
a cathode; and
an organic layer provided between the anode and the cathode, wherein the organic layer comprises the composition according to any one of claims 1 to 6.

10. The organic electroluminescence device according to claim 9, wherein
the organic layer comprises a plurality of organic layers, and at least one of the plurality of organic layers comprises the composition according to any one of claims 1 to 6.

11. The organic electroluminescence device according to claim 9 or 10, wherein
the organic layer comprises an emitting layer, and the emitting layer comprises the composition according to any one of claims 1 to 6.

12. The organic electroluminescence device according to claim 11, wherein the emitting layer further comprises a luminescent material.

13. The organic electroluminescence device according to claim 12, wherein
the emitting layer comprises a phosphorescent material as the luminescent material, and the phosphorescent material is an ortho-metalated complex of a metal atom selected from the group consisting of iridium, osmium and platinum.

14. The organic electroluminescence device according to any one of claims 9 to 13, further comprising a hole transporting layer between the anode and the emitting layer.

15. The organic electroluminescence device according to any one of claims 9 to 14, further comprising an electron transporting layer between the cathode and the emitting layer.

16. An electronic device comprising the organic electroluminescence device according to any one of claims 9 to 15.
